# EUROPEAN PATENT SPECIFICATION

(11) **EP 4 061 960 B1**
(45) Date of publication and mention of the grant of the patent: **01.05.2024**
(21) Application number: 20804562.5
(22) Date of filing: 16.11.2020
(51) Int. Cl.: C12Q 1/6806, C12N 15/10, C12Q 1/6897

(54) **NOVEL METHOD FOR POOLED INTRON TAGGING FOR REAL TIME DRUG SCREENING**
NEUES VERFAHREN ZUR GEPOOLTEN INTRON-MARKIERUNG FÜR ECHTZEIT-WIRKSTOFFSCREENING
NOUVEAU PROCÉDÉ D'ÉTIQUETAGE D'INTRONS GROUPÉS POUR CRIBLAGE DE MÉDICAMENTS EN TEMPS RÉEL

(30) Priority: 22.11.2019 EP 19211077
(43) Date of publication of application: 28.09.2022
(73) Proprietor: CeMM - Forschungszentrum für Molekulare Medizin GmbH, 1090 Wien (AT)
(72) Inventor: KUBICEK, Stefan, 1100 Vienna (AT); REICHER, Andreas, 1090 Wien (AT)
(74) Representative: Vossius & Partner Patentanwälte Rechtsanwälte mbB
(86) International application number: PCT/EP2020/082295
(87) International publication number: WO 2021/099273

(56) References cited:
- WO-A2-2019/210268
- YEVGENIY V. SEREBRENIK ET AL: "Efficient and flexible tagging of endogenous genes by homology-independent intron targeting", GENOME RESEARCH, vol. 29, no. 8, 25 June 2019 (2019-06-25), pages 1322-1328, XP055762046, US ISSN: 1088-9051, DOI: 10.1101/gr.246413.118 cited in the application

## Description

The present invention relates to a method for monitoring the effect of an environmental factor on the proteome of a cell.

While most currently available pharmacological agents, including small molecule pharmaceuticals or pharmacologically active biologics act as inhibitors of enzymes or as modulators of receptors and transporter, drugs may also exert other functions, like (but not limited to) the inhibition or induction of protein-protein interactions and the stabilization or degradation of target proteins.

Currently available methods for the unbiased discovery and for the elucidation of biological/pharmacological functions of bioactive compounds ("mechanisms of action") and/or some of the known screening methods of substances for their potential use pharmaceuticals are based on the monitoring of the biological and/or pharmacological effects on proteomes and transcriptomes; see, inter alia, Rix (2009) Nat Chem Biol 5, 616-24; Martinez Molina (2013) Science 341, 84-7; Savitski (2014) Science 346, 1255784; Drewes (2015) Trends Biotechnol 36, 1275-1286; Huber (2015). Nat Methods 12, 1055-7; Subramanian (2017). Cell 171, 1437-1452 e17; or Lamb (2006) Science 313, 1929-35.

Yet, costs and sample preparation requirements associated with these methods preclude their application in large scale screenings and/or they preclude the use of these methods on a large number of drugs/drug candidates at multiple concentrations and/or time points of assessment. Furthermore, other high-content screening approaches that monitor drug effects on cell morphology, as disclosed in Bray (2016) Nat Protoc 11, 1757-74 and/or protein localization approaches by microscopy, for example by staining of fluorescent-tagging approaches, are hampered by the fact that these methods merely allow the monitoring of one or of only a few selected proteins.

The prior art saw in this context approaches in which fluorescently tagged reporter cells are generated either by overexpression to non-physiologic levels, by targeting a single gene with a homologous recombination template. Also "genetrap" approaches have been applied in this context; see, e.g. Morin (2001) Proc Natl Acad Sci U S A 98, 15050-5). Yet, such approaches are limited by integration site biases. Yet, these "genetrap virus approaches" employ viral constructs in order to generate tagged cell pools. Since the employed viruses have tremendous integration site biases, namely in the first intron, some genes are targeted much more efficiently by these vial constructs than others. Furthermore, there are no means in these approaches to select specific gene sets or specific introns to be targeted.

Serebrenik and colleagues proposed a tagging technology of endogenous genes by homology-independent intron targeting, whereby intron-based protein trapping with homology-independent repair-based integration of a generic donor was combined, see Serebrenik (2019) Genome Research 29, 1322-28. The corresponding approach is based on homology-independent CRISPR-Cas9 editing to place a fluorescent tag as a synthetic exon into introns of individual target genes has been described by combining a generic sgRNA excising a fluorescent tag flanked by splice acceptor and donor sites from a generic donor plasmid with co-expression of a gene-specific intron-targeting sgRNA. Based on the fact that this technology employs generic donors, it is speculated that this technology would enable the generation of multiple fusion cell lines but that this would require the cloning of additional intron-targeting sgRNAs. Yet, from the technology as provided by Serebrenik, an efficient way to determine which cell expresses which protein is not feasible.

Accordingly, there is a need in the art to provide for means and methods for a characterization of factors influencing individual proteins comprised in the whole proteome whereby the whole proteome or at least a substantial part thereof is or can be assessed.

The technical problem is solved by the embodiments as characterized in the claims and as provided herein.

Disclosed is a method for monitoring the effect of an environmental factor on the proteome or parts thereof of a cell, the method comprising the steps of:
(a) selecting introns to be targeted in the genome of a cell;
(b) identifying guide RNA (gRNA) sequences suitable for inserting a tag in the selected introns in the genome of the cell;
(c) cloning identified gRNA sequences and tag sequence into transduction vectors;
(d) contacting a population of the cell with said vectors of (c) to integrate the tag of (b) into selected introns;
(e) exposing cell population to environmental factor; and
(f) monitoring the effect of the environmental factor on the proteome based on the detection of the tag prior to exposure of the cell population to the environmental factor and subsequent to exposure of the cell population to the environmental factor.

The present invention relates to a method for monitoring the effect of an environmental factor on the proteome or parts thereof of a cell, the method comprising the steps of:
(a) selecting introns to be targeted in the genome of a cell;
(b) identifying guide RNA (gRNA) sequences suitable for inserting a tag in the selected introns in the genome of the cell;
(c) cloning identified gRNA sequences and tag sequence into vectors;
(d) contacting a population of the cell with said vectors of (c) to integrate the tag of (b) into selected introns;
(e) exposing cell population to environmental factor; and
(f) monitoring the effect of the environmental factor on the proteome based on the detection of the tag prior to exposure of the cell population to the environmental factor and subsequent to exposure of the cell population to the environmental factor
wherein in step (c) the identified gRNA sequences are cloned into a transduction vector and the tag sequence is cloned into a minicircle DNA.

Accordingly, within the present invention, the tag sequence may be cloned into a minicircle DNA which is then used as vector for transduction. Accordingly, the term "cloning identified gRNA sequences and tag sequence into transduction vectors" as used herein is to be understood as comprising the cloning into a minicircle DNA as a vector.

As shown in the appended examples, the methods of the present invention comprise a step of identifying gRNA sequences suitable for inserting a tag in introns in the genome of a cell. In the methods of the present invention, it is preferred that a cell comprised in a population of cells that is to be tagged receives a single tag. The general principle of intron tagging is described by Serebrenik et al. (2019), loc cit. The strategy of Serebrenik et al. relies on a single generic sgRNA excising a single fluorescent tag flanked by splice acceptor and donor sites from a generic donor plasmid, which is co-expressed with a single gene-specific intron-targeting sgRNA specifying the single integration site.

However, as shown in the appended examples, the methods of the present invention generate an intron-tagged cell pool that is then used to characterize/monitor effects of an environmental factor on the proteasome or parts thereof of a cell type. This is achieved by tagging cells in a population of cells of the same cell type, whereby each cell receives a single tag. The population thus comprises cells tagged at different genomic sites, providing as a whole a tagged proteome or tagged parts thereof. Accordingly, and in contrast to the technology as provided by Serebrenik et al. (2019), loc cit., the present invention provides for means and methods wherein the whole proteome (or at least substantial parts thereof) can be monitored in one individual cell population. Therefore, the present invention provides for a "one shot" analysis of the whole proteome (or substantial parts thereof). As such, the present invention, for the first time, allows the analysis of the whole proteome (or at least substantial parts thereof) in one experiment by using different gRNA sequences for a multitude of tagged proteins combined with *in situ* sequencing. In this regard, the inventors found that the use of a sequencing-enabling vector that expresses the gRNA as part of the transcript that can be detected by *in situ* sequencing, such as a CROPseq vector, as a transduction vector allows the identification of the individual gRNA sequence, which corresponds to the tagged protein in each clone in the pool, e.g. using an imaging technique such as microscopy.

While most currently available pharmacological agents, including small molecule pharmaceuticals or pharmacologically active biologics act as inhibitors of enzymes or as modulators of receptors and transporters, drugs may also exert other functions, like (but not limited to) the inhibition or induction of protein-protein interactions and the stabilization or degradation of target proteins. In context of this invention, a scalable strategy to discover in real time the effects drugs exert on levels and subcellular localizations of a large subsets of the proteome is provided. Illustratively for the present invention, CRISPR-Cas9 based intron tagging was employed to generate cell pools expressing hundreds of GFP-fusion proteins at endogenous levels, monitor drug effects on protein levels and localization by time-lapse microscopy, and identify targeted introns by in situ sequencing. This is also documented in the appended figures and examples. From the pool of tag-positive cells (here illustratively GFP positive cells), more than 500 individual clones are isolated and these positive cells where analyzed/imaged by fluorescence microscopy in order to reveal the subcellular protein localization of many proteins for which the subcellular protein localization had not been previously characterized. Furthermore, the inventive pool of cells may also be used to study protein dynamics in response to various metabolic perturbations either in an arrayed or pooled format and strategies to identify individual drug-responsive clones in the pool are provided.

As disclosed herein, the present invention provides for methods for monitoring the effect of an environmental factor on the proteome or parts thereof of a cell. In context of this invention, the term "part(s) of the proteome" relates to a substantial part of the proteome, i.e. at least 100, at least 200 at least 300 at least 400 at least 500, at least 600, at least 700 and more preferably at least 900 expressed genes (coding for proteins).

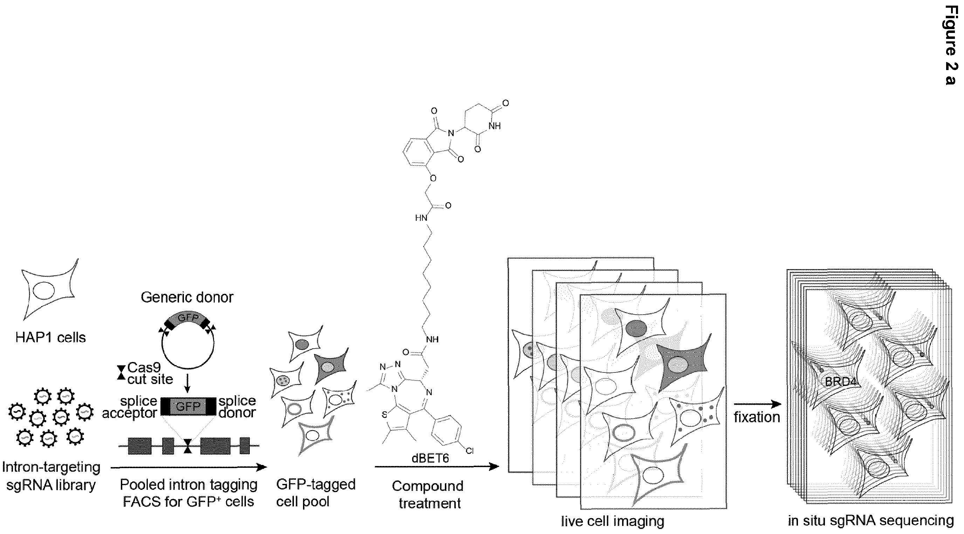

In particular and in contrast to the prior art, in particular Serebrenik et al., the invention provided herein allows scalability to enable pooled protein tagging of a multitude of metabolic enzymes and epigenetic modifiers. As shown in the appended Examples, more than 900 metabolic enzymes were targeted. Exposing the GFP-tagged cells to compounds to monitor drug effects on the localization and levels of hundreds of proteins in real time in a pooled format, followed by identification of responding clones by in situ sequencing of the expressed intron-targeting sgRNA that corresponds to the tagged protein, as shown in Fig. 2a, represents a major advantage over methods of the prior art.

It is preferred that the methods of the present invention further comprise a sequencing step subsequent to step (d).

As detailed above, the sequencing step allows the association of individual cells with tagged proteins by sequencing the individual gRNA. This may be achieved by sequencing the gRNA insert while it is not necessary to sequence the protein directly. This can either be done on whole population level or based on expressed proteins, for example subsequent to a cell sorting step based on the expressed tag. Accordingly, in the methods of the present invention, the gRNA insert, or a part thereof, of (a) cell(s) of the population is sequenced in the genome of said cell(s) or in the transcriptome of said cell(s).

In a further preferred embodiment, the sequencing step is subsequent to step (f) of the methods of the invention.

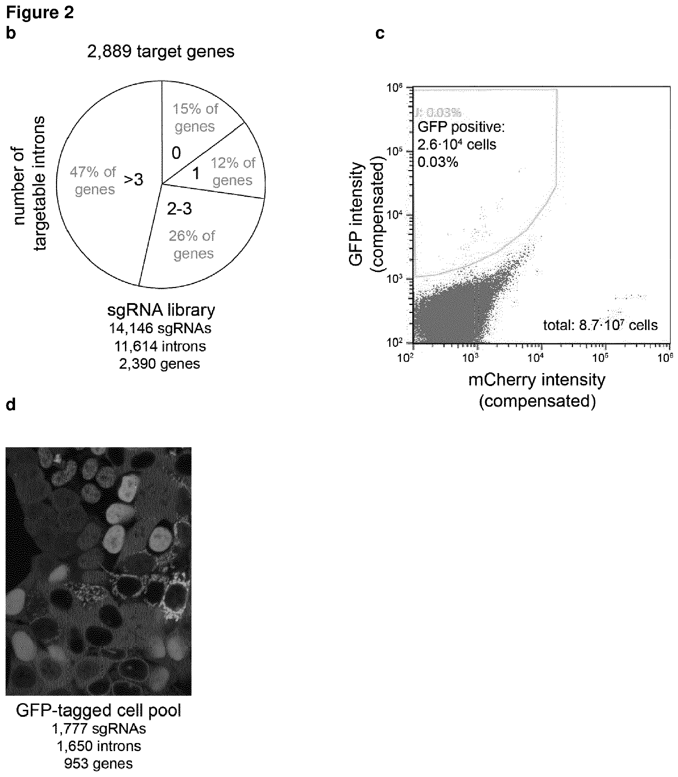

Sequencing of the gRNA insert in the transcriptome preferably further comprises a step of reverse transcription and the use of a sequencing vector as transduction vector. An exemplary vector suitable for sequencing is a Crop-Seq vector. In an exemplary embodiment, the procedure may be as in Fig. 2c. The method may be further adapted by using in situ sequencing using the Illumina NextSeq 500/550 kit v2, which provides a two-color system compatible with the GFP expressing cells.

As shown in the appended examples, the introns to be targeted are selected based on the reading frame of the upstream exonic sequence, wherein the to be inserted sequence is in-frame with the exonic sequence. As shown in Figure 2b, designing the intron targeting library required the inventors to analyze the target gene set regarding the number of available introns (Fig. 2b) and whether the introns were in the right reading frame (only one out of 3 reading frames is suitable to generate functional GFP integrations). Based on these criteria 14,146 sgRNA sequences for 11,614 introns in 2.390 genes were generated. Furthermore, the introns to be targeted can be comprised in genomic sequences of metabolic enzymes, chromatin proteins, kinases, genes coding for proteins in the ubiquitin/proteasome pathways, transcription factors, ion channels, transporters, receptors or at least one intron per protein coding gene in the genome can be targeted.

Design of sgRNA sequences is further based on cutting efficiency. Thus, in a further embodiment, the gRNA sequences suitable for inserting a tag in the selected introns in the genome of the cell are identified according to cas9 cutting efficiency or Cpf1 cutting efficiency or Cas12b cutting efficiency. Additionally, or alternatively, the gRNA sequences suitable for inserting a tag in the selected introns in the genome of the cell are identified according to their occurrence in the genome of the cell, preferably wherein the occurrence is 1.

The vector further encodes for a tag that is to be inserted into the intron. The tag can be any tag allowing detection subsequent to integration or expression. Preferably, the tag is a fluorescence tag (preferably green fluorescent protein (GFP or enhanced GFP) or yellow fluorescent protein (YFP), or red fluorescent protein (RFP) or a tag suitable for detection by covalent (e.g. Halo tag, Clip tag, Snap tag) or non-covalent (e.g. Strep-tag, HA tag, dTag) binding to a detection reagent enabling detection by microscopy by fluorescence or luminescence.

Once the sgRNA sequences and the tag sequences have been selected and cloned, the library of sgRNA vectors is contacted with a population of a cell to integrate the tag of into selected introns.

To ensure that each cell gets infected only with a single vector and thus only one intron-targeting sgRNA, cells are preferably infected at a multiplicity of infection of below 1, preferably 0.8, 0.6, 0.4, more preferably 0.2. The term "multiplicity of infection" is to be understood as meaning that only 80%, 60%, 40% or 20%, respectively, of the cells are infected.

In order to select for infected cells, a selection marker can be comprised in the vector. An exemplary marker is the puromycin selection marker also present on the vector, e.g. the CROP-seq vector.

In an exemplary method, transient transfection can subsequently be used to introduce a plasmid for expression of Cas9 (that would introduce a cut specifically in one intron as specified by the sgRNA/gRNA previously introduced to the same cell with the CROP-seq vector) and a generic sgRNA/gRNA. A second plasmid can also be introduced that acts as a generic donor plasmid that provides the tag sequence, for example an EGFP sequence, to be integrated into the intron. This plasmid contains a Cas9 cut-site (targeted by the generic sgRNA sequence present on the Cas9 plasmid), a splice acceptor site, tag sequence (e.g. EGFP), a splice donor site and another Cas9-cut site (targeted by the generic sgRNA sequence present on the Cas9 plasmid). As an alternative to the generic donor plasmid with two Cas9 cut-sites, minicircle DNA that does not comprise a plasmid backbone and a single Cas9 cut-site, a splice acceptor site, tag sequence (e.g. GFP or EGFP) and a splice donor site may be used. When using a minicircle, the intron tagging efficiency is increased, due to the lack of a plasmid backbone that can get integrated at the intronic integration site instead of the tag sequence containing fragment. The methods may further comprise selection for cells that are successfully transfected (e.g. blasticidin marker on the Cas9 plasmid) and expansion of the cells, for example over a period of 5 days.

The methods of the present invention may further comprise a step of separating tagged cells from non-tagged cells. The separation method depends on the tag that is used. In a preferred embodiment, the cells are fluorescence tagged and the cells are separated using FACS. Accordingly, FACS or an alternative separation method can be used to sort out targeted cells. In addition, tagged proteins can be selected according to expression levels. That is, all proteins are expressed at endogenous levels, and some proteins are expressed to very low levels. To differentiate between expression levels, a further parameter may be used (e.g. a further channel during selection), e.g. for cell-specific background fluorescence and sort cells that are enriched for the tag, for example GFP or EGFP (Fig. 2c).

The sorted pool of tagged cells or the unsorted pool comprising tagged cells may further be characterized using a suitable method based on the introduced tag. For example, protein expression, protein localization, surface expression, protein-protein interaction, protein stability, and/or protein mobility may be monitored using a suitable detection method.

As such, also disclosed is a population of cells comprising multiple cells each comprising an inserted tag sequence in an intron of said cell, wherein the tag is inserted in-frame with the preceding exonic sequence and wherein the intron into which the tag is inserted is different between cells.

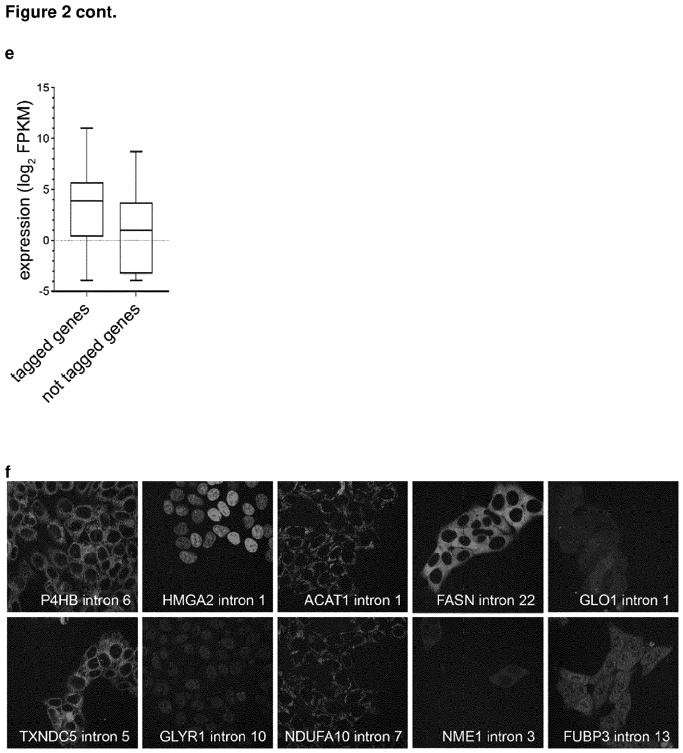

As detailed above, the cells may also be characterized by sequencing. In one exemplary embodiment, the intron tagged cell pool can be characterized by PCR amplifying the integrated sgRNA sequence from genomic DNA, next generation sequencing and mapping back to the sequences in the designed sgRNA library. This way it was determined that more highly expressed genes were more likely to be successfully tagged (Fig. 2e).

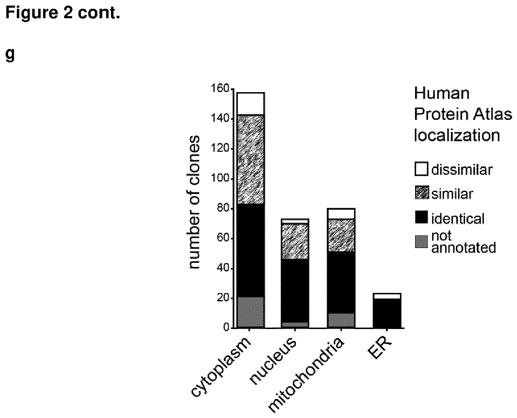

The intron tagged cell pool can be further characterized by diluting to single cells and growing them up to large colonies on a 96-well plate. These single-cell derived clones can be characterized by imaging (Fig.2f.) to identify the targeted protein again by PCR amplifying the integrated sgRNA sequence from genomic DNA, next generation sequencing and mapping back to the sequences in the designed sgRNA/gRNA library. This way it could be confirmed that the vast majority of cells indeed harbor only a single tagged protein. Furthermore, it was observed that the vast majority of protein locations in the clones corresponded to those identified by antibody-based staining of the targeted protein in the Human Cell Atlas (Fig. 2g).

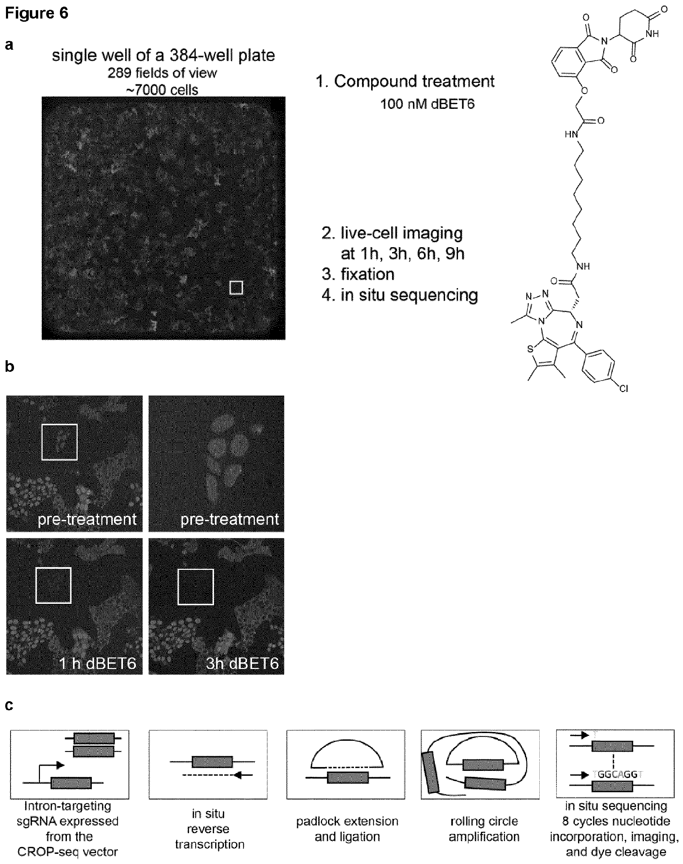

It is thus an object of the present invention to monitor effects of environmental factors on the proteome or parts thereof of a cell. The environmental factor may be selected from radiation, a chemical compound, a biological compound, temperature, nutrient depletion, ion concentrations or combinations thereof. In an exemplary embodiment, the method may comprise plating of cell pools at conditions of approximately 7,000 cells per well in a 384-well plate (Fig. 6a). While the method works for a single cell per tagged protein, it is preferred to have at least 5 cells per tagged protein for robustness. An image from the cell pool before compound treatment is taken. Subsequently, the cells are exposed to the environmental factor, for example by adding a chemical compound. The chemical compound may be a drug, a small molecule, an siRNA, an shRNA/sgRNA virus, a protein. In one embodiment, the environmental factor may be at different intensities/concentrations for different cell populations, for example to obtain dose-response curves. Subsequently, at least one further image is taken. However, multiple images may be taken in order to follow the effect on the time scale. For example, images may be taken after 1h, 3h,6h,9h, but this timeframe can be freely adjusted (Fig. 6b).

Within the present invention, the cell may be a HAP1 cell, K562 cell, HeLa cell, KBM7 cell, BT474 cell, MG-63 cell, SKNAS cell, A427 cell, A375 cell, A498 cell, RCH-ACV cell, HEK293T cell, A673 cell, SK-N-MC cell, A549 cell, SKMES1 cell, NCIH727 cell, THP1 cell, NB4 cell, MOLM13 cell, KASUMI-1 cell, HEL cell, NB-4 cell, HL-60 cell, RS4-11 cell, MOLT7 cell, aTC1 cell, bTC3 cell, Min6 cell or another cell line, preferentially an adherent, non-migratory cell line .

Also disclosed is a population of cells comprising multiple cells each comprising an inserted tag sequence in an intron of said cell, wherein the tag is inserted in-frame with the preceding exonic sequence and wherein the intron into which the tag is inserted is different between cells. Accordingly, the present invention provides for a novel and inventive cell population that is characterized in that said cell population and that comprises multiple cells each individually tagged in one intron sequence of a gene coding for an (expressed) protein. As such the population of cells provides a novel and inventive tool for whole proteome analysis as well as for a valuable tool for drug screenings on a cellular basis. Also disclosed is a kit comprising said novel and inventive cell population. Such a kit is also particularly useful as means for drug screenings, drug evaluations, treatment monitoring, as research tool for basic sciences. Further uses of the inventive cell population are within the capabilities of the skilled artisan.

The disclosed population of cells is obtained by a method comprising the steps of:
(a) selecting introns to be targeted in the genome of a cell;
(b) identifying guide RNA (gRNA) sequences suitable for inserting a tag in the selected introns in the genome of the cell;
(c) cloning identified gRNA sequences and tag sequence into vectors, preferably wherein the gRNA sequences are cloned into transduction vectors and the tag sequence is cloned into a donor plasmid or minicircle DNA;
(d) contacting a population of the cell with said vectors of (c) to integrate the tag of (b) into selected introns; and
(e) obtaining the population of cells.

The population of cells disclosed herein and, in particular, as obtained by the method of the invention, can be used e.g. for screening purposes, in particular drug screening. For example, the population of cells may be used in a method for screening a drug for its effect on the proteome of the cell population. This may be used, inter alia, in the screening of a drug suitable for downregulating/upregulating a specific protein or group of proteins that is/are part of the proteome of the population of cells.

The invention is further illustrated by the following non-limiting figures and examples:

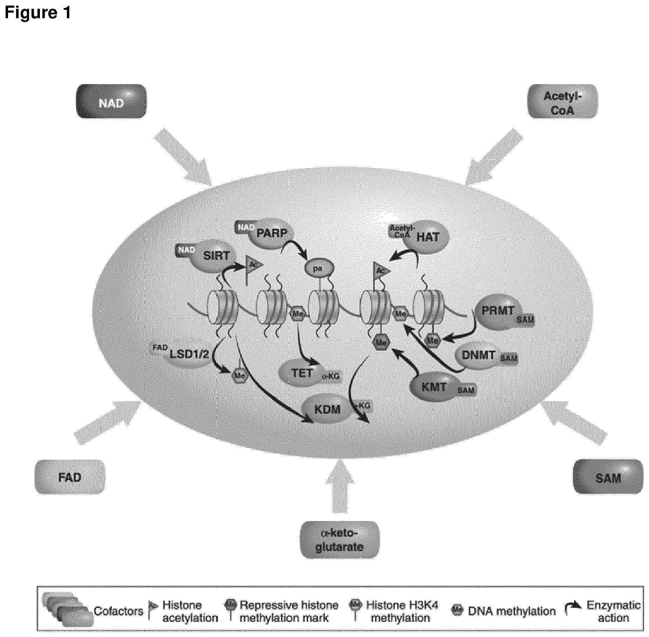

**Figure 1****: Many cytoplasmic and mitochondrial metabolic enzymes are found in the nucleus.** The subcellular localization of some metabolic enzymes changes during the cell cycle or in response to perturbations. Protein localization of can be studied by immunofluorescence staining or by endogenously tagging proteins on the N- or C-terminus. (Figure from Berger and Sassone-Corsi (2016), Cold Spring Harb Perspect Biol 8:a019463)
**Figure 2****: Pooled GFP intron-tagging of metabolic enzymes, a.** Schematic outline of the approach. **b.** Identification of targetable introns within metabolic genes. **c.** FACS sorting of clones with successful GFP-tagging by signal enrichment over background mCherry intensity. **d.** Representative image of sorted GFP-tagged cell pool. **e.** Comparison of RNA-seq expression in HAP1 cells between genes for which GFP-tagged cells could be isolated and genes that were targeted in the sgRNA library but did not result in successful clone isolation. **f.** Representative images of individual clones isolated by single cell dilution and identified by sgRNA NGS. **g.** Comparison of localizations of 335 individually isolated clones to localization annotations in the Human Protein Atlas.

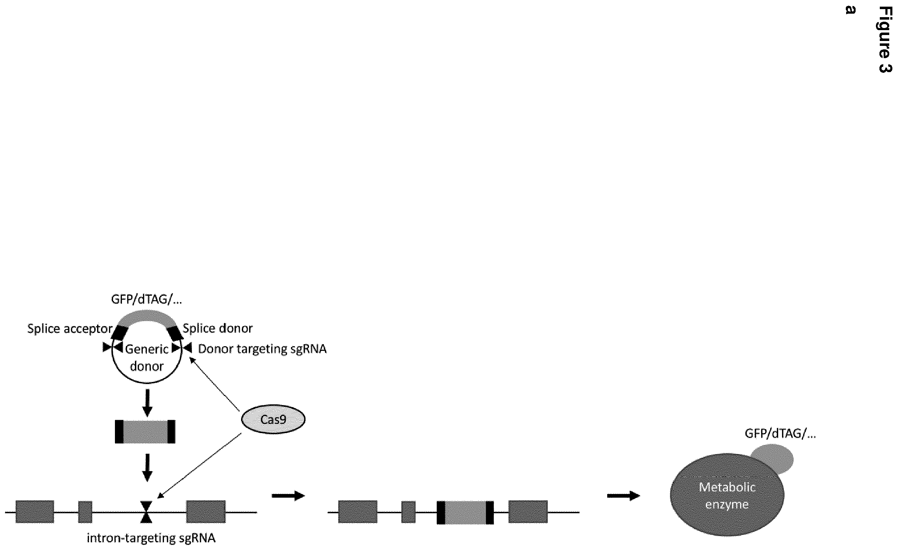

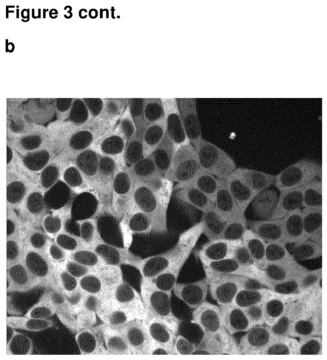

**Figure 3****: Knock-in of a protein tag using an intron targeting strategy, a.** Intron-tagging strategy with an intron targeting sgRNA, and a generic sgRNA targeting a donor plasmid targeting that provides a synthetic exon with the protein tag. This synthetic exon without homology arms is integrated into the intronic target site specified by the specific sgRNA via more NHEJ. This is a scalable targeting strategy as the same generic donor can be applied for all targets. b. Representative image confirming successful targeting of intron 26 in MTHFD1 with 2.2% efficiency.

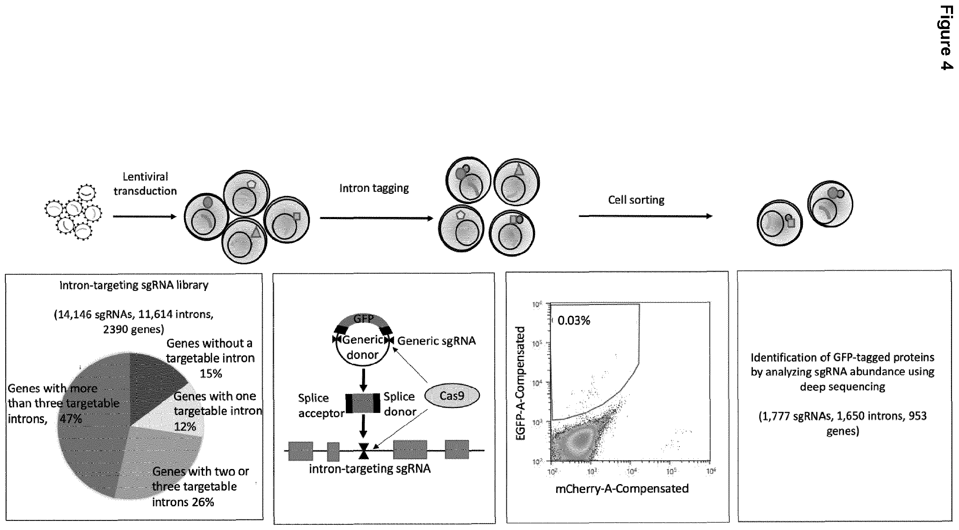

**Figure 4****: Pooled protein-tagging of metabolic enzymes and epigenetic modifiers**

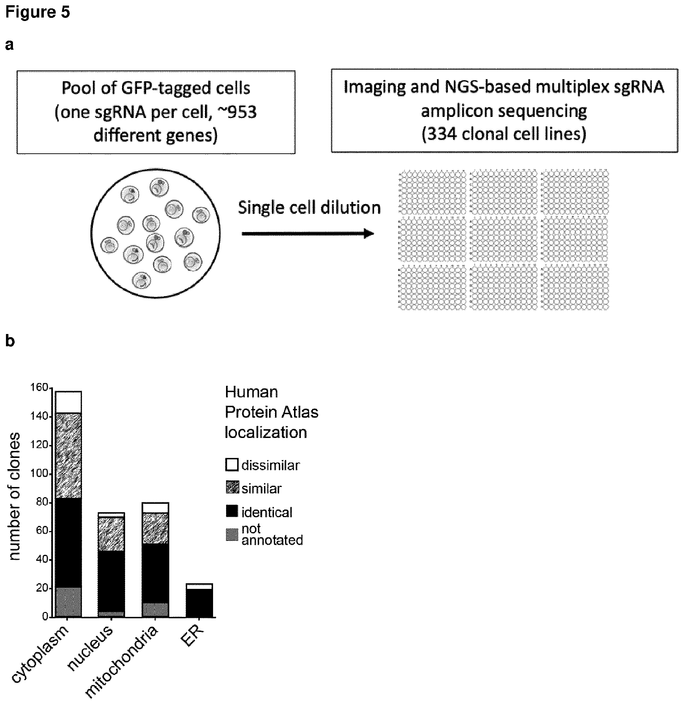

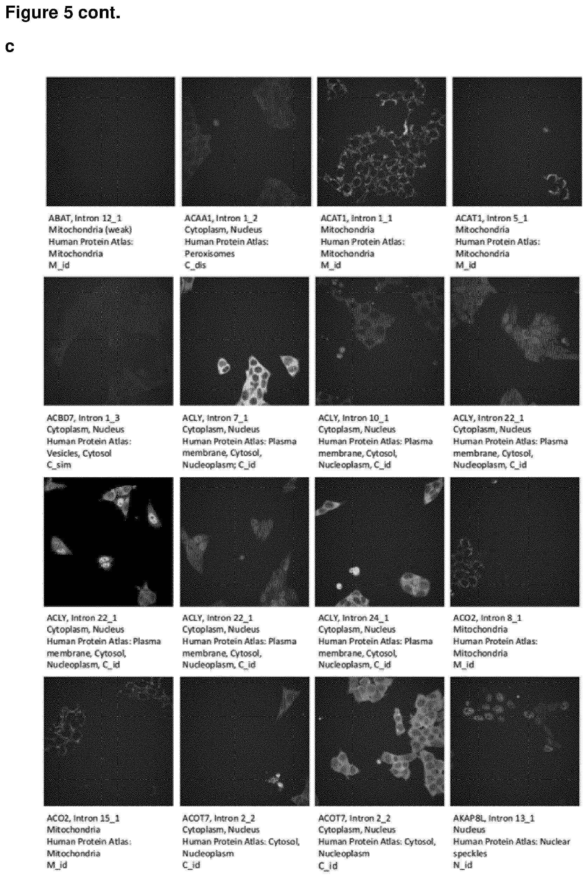

**Figure 5****: Isolation of 334 clonal cell lines and comparison of subcellular protein localization to The Human Protein Atlas. a.** Single cell cloning approach by single cell dilution to 96-well plates. **b.** Comparison of localizations of 335 individually isolated clones to localization annotations in the Human Protein Atlas. c. Representative images for the first 16 clones alphabetically

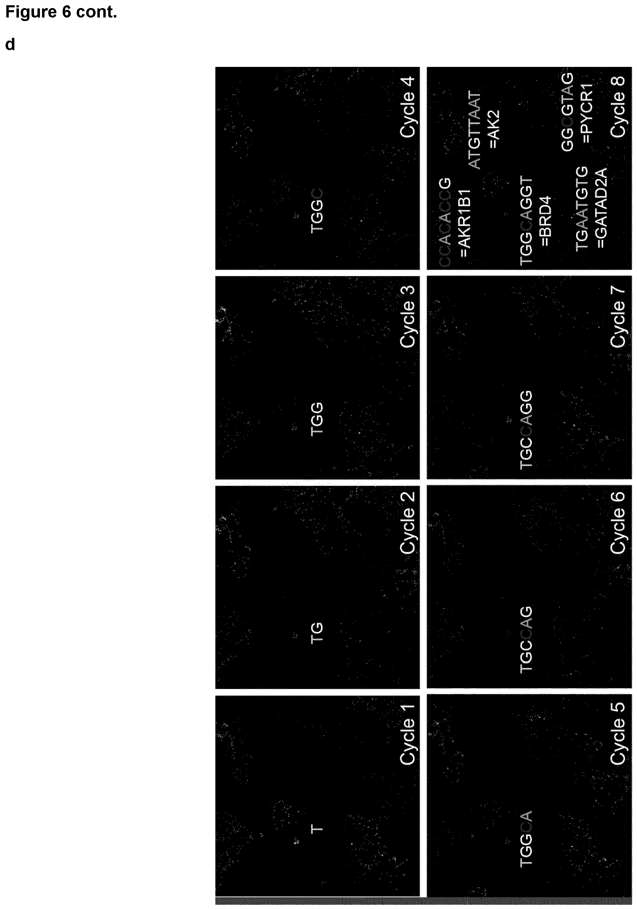

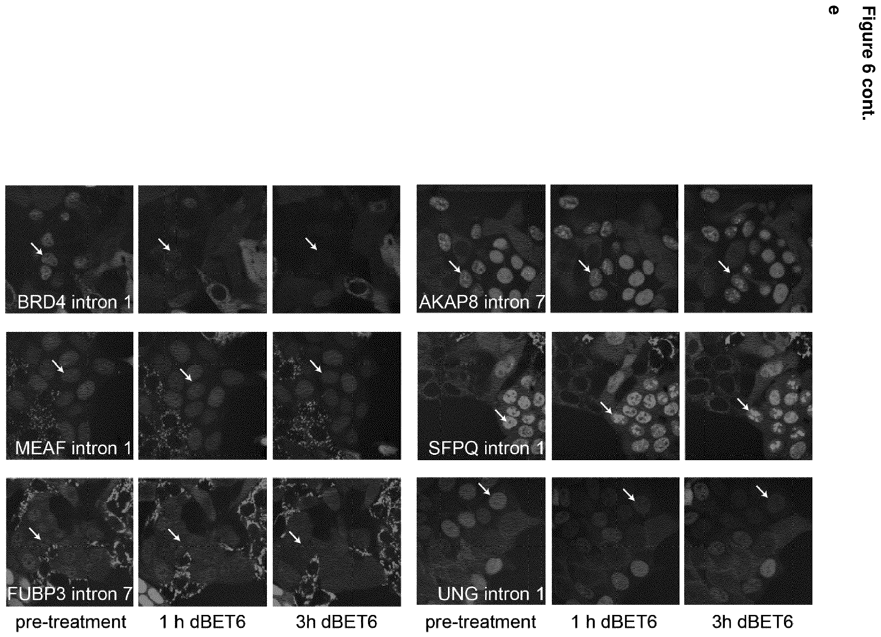

**Figure 6****: Compound screening on cell pools followed by in situ sequencing enables the detection of protein-specific compound effects, a.** Stitched image of 289 fields of view representing an entire well on a 384-well plate containing approximately 7,000 individual cells. **b.** Identification of a clone with rapid loss of GFP signal following treatment with 100 nM dBET6, while neighboring clones are unaffected. **c.** Outline of the in situ sequencing approach. **d.** Images from 8 cycles in situ sequencing of the area shown in panel b. **e.** Selected images for clones showing localization changes following dBET6 treatment.

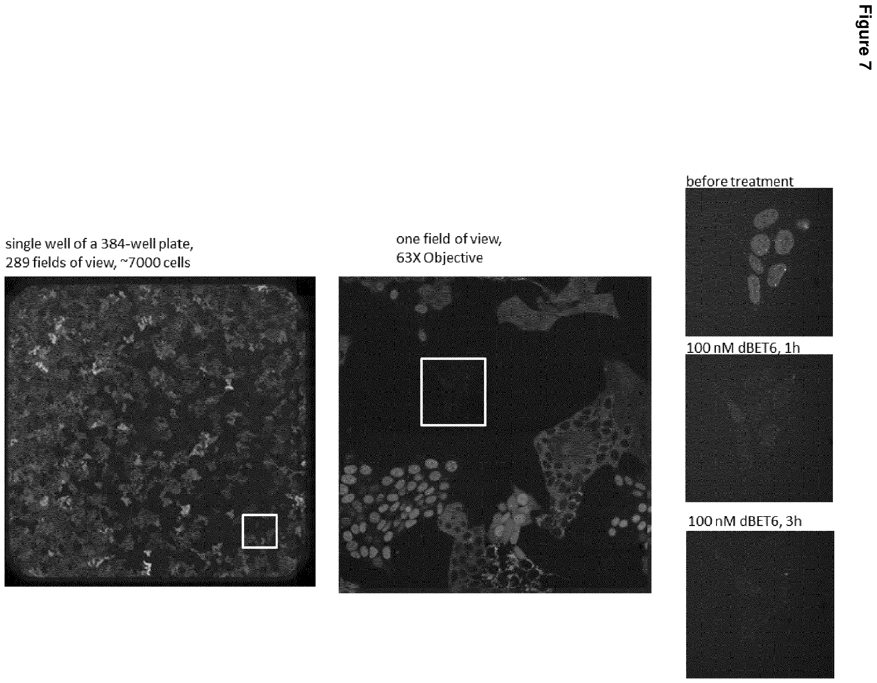

**Figure 7****: Pooled screening approach to study drug effects on protein levels and localization.**

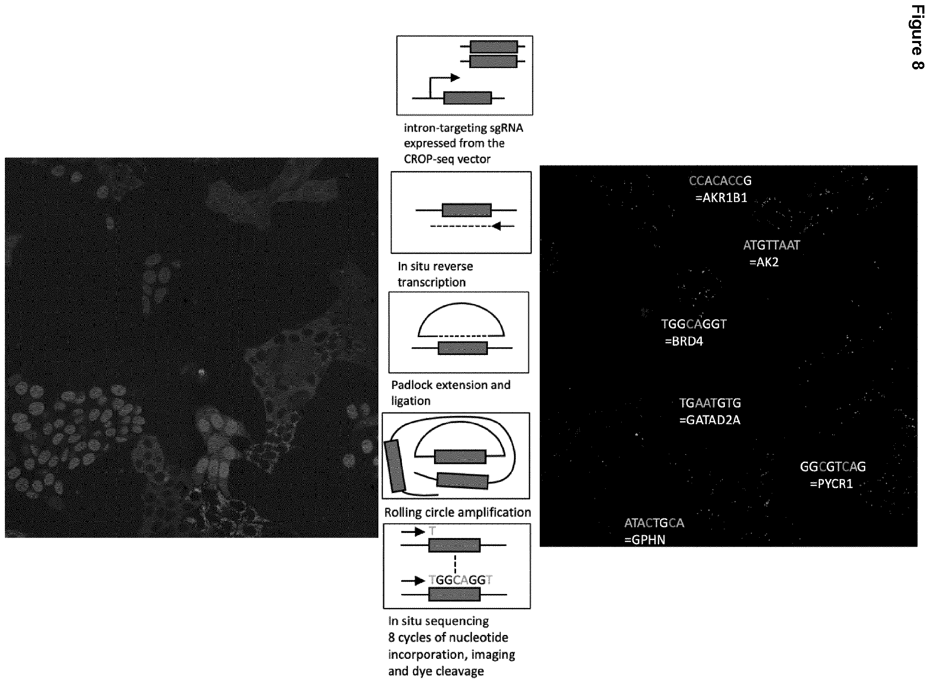

**Figure 8****: In situ sequencing to identify the tagged protein in individual clones.**

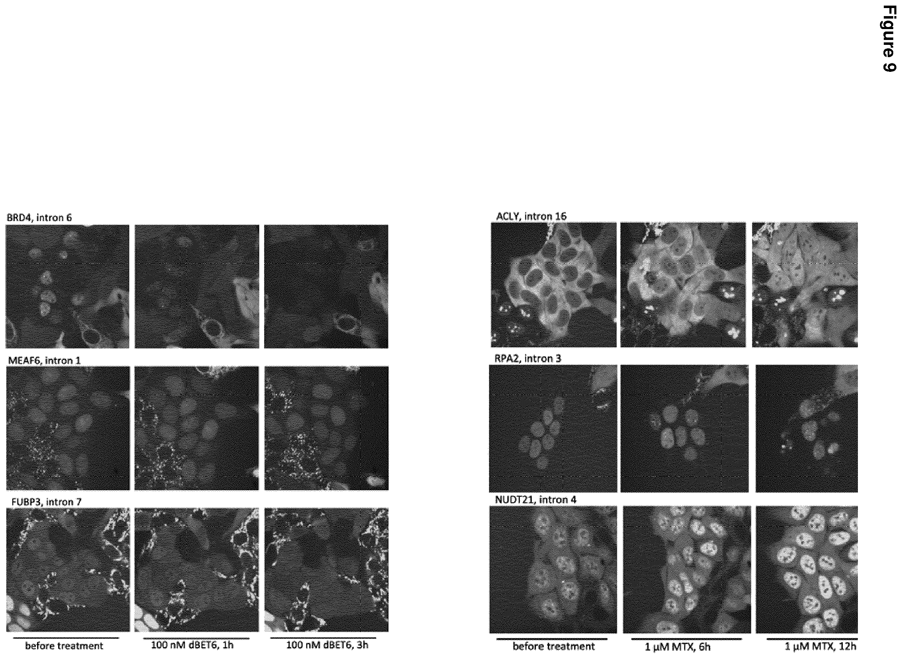

**Figure 9****: Effects on protein localization and levels induced by the BRD4 degrader dBET6 and methotrexate.**

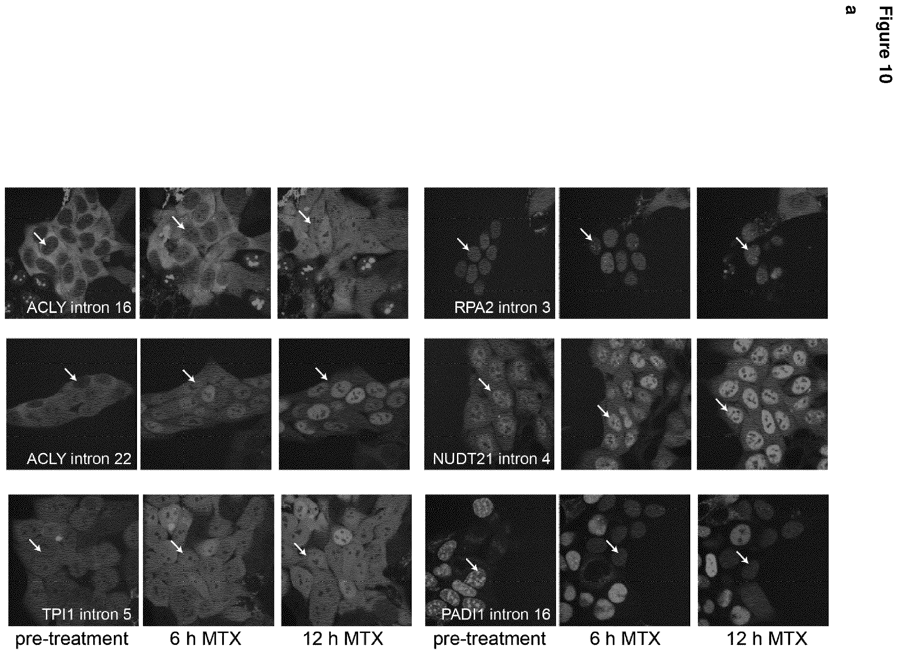

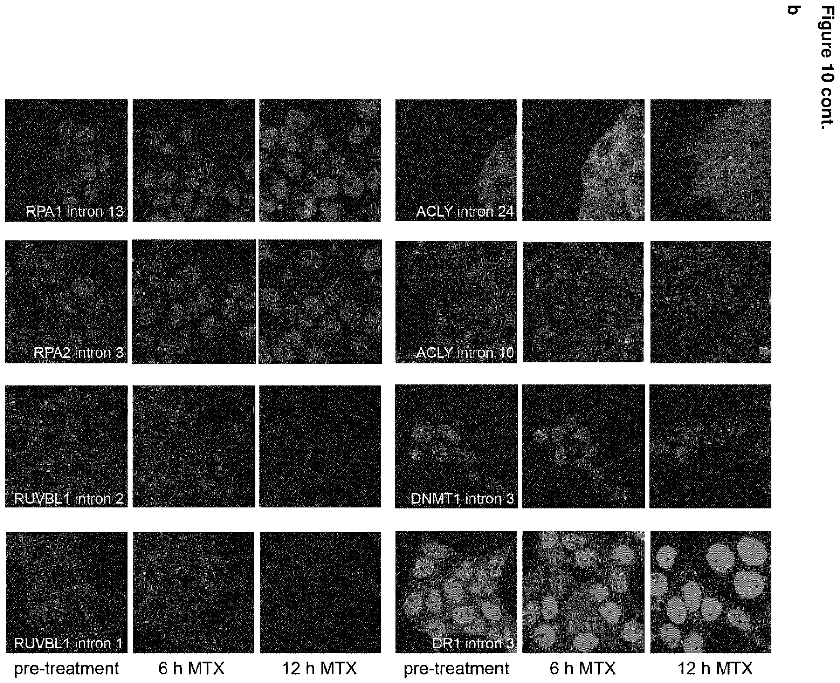

**Figure 10** **Time-dependent changes in protein localization and levels following treatment with 1 µM methotrexate. a.** Selected images showing changes following methotrexate treatment in cell pools, annotated by clone identification from in situ sequencing. **b.** Selected images showing changes in 335 arrayed clones exposed to methotrexate.

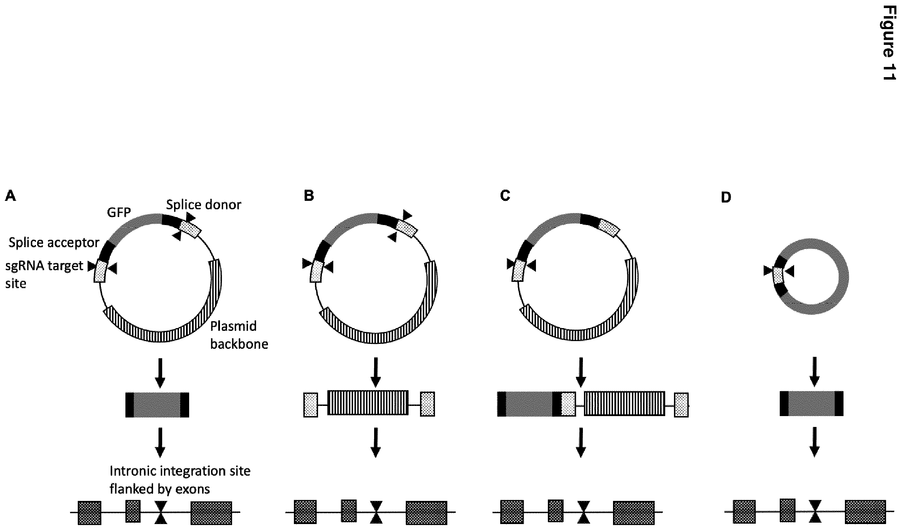

**Figure 11** **A.** The GFP donor plasmid is cut at two sgRNA targeting sites followed by integration of a fragment containing GFP flanked by splice acceptor and splice donor sites. **B.** The plasmid backbone gets integrated instead of the GFP containing fragment. **C.** The GFP donor plasmid is cut only once and the entire plasmid gets integrated. **D.** A minicircle has only one sgRNA target site and no plasmid backbone can get integrated.

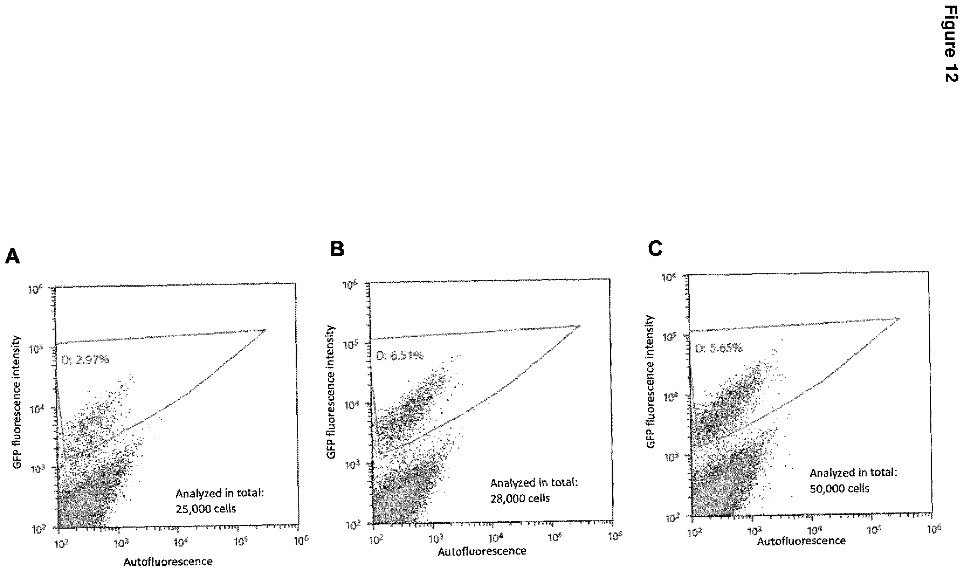

**Figure 12** Tagging rates when targeting the CANX gene and **A)** the conventional GFP donor plasmid, **B)** the same amount of minicircle DNA and **C)** one third the amount of minicircle DNA.

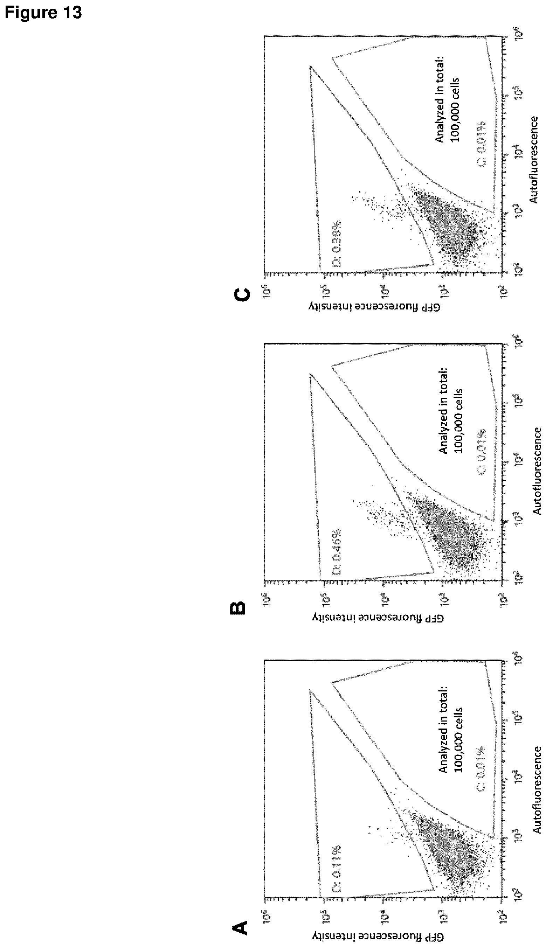

**Figure 13** Tagging rates when targeting the CANX gene and **A)** the conventional GFP donor plasmid, **B)** the same amount of minicircle DNA and **C)** one third the amount of minicircle DNA in cell populations that were not enriched for transfected cells.

### Example 1: Employed methods

### Generation of an intron-targeting saRNA library

To design an intron-targeting sgRNA library for metabolic enzymes and epigenetic modifiers a list of 2,889 genes was generated by combining a published list of all classic metabolic enzymes (see, Corcoran (2017) Am J Physiol Renal Physiol 312, F533-F542), most genes in a human CRISPR metabolic gene knockout library (see; Birsoyv (2015) Cell 162, 540-51) as well as genes annotated with the GO terms "Histone modification", "DNA methylation" or "DNA demethylation". Then, the Ensembl BioMart data mining tool was used to obtain chromosomal coordinates of introns of the primary transcripts of those genes and only those introns were selected where integration of the donor plasmid does not lead to frameshift mutations after splicing, since the donor plasmid starts with a full codon and is not compatible to all exon-exon junctions. Using Ensembl BioMart this filtering was done by only selecting introns that are preceded by an exon with the attribute "End phase = 0". The GuideScan (Perez, 2017, Nat Biotechnol 35, 347-349) was then used to obtain the top 20 guides for each selected intronic region based on the GuideScan cutting efficiency score. Those 20 guides were then ranked based on a combined on- and off-target score using the scores provided by GuideScan. For genes that have only one intron that can be targeted, up to three sgRNAs per intron were selected, for genes with two or three introns that can be targeted, up to 2 sgRNAs per intron were selected and for genes that have more than three introns that can be targeted, the top ranked sgRNA of each intron was selected. Using that strategy, 14,049 sgRNAs targeting 11,614 introns of 2,387 genes were selected. In addition, 75 non-targeting sgRNAs from the human Brunello CRISPR KO library (Doench, 2016, Nat Biotechnol 34, 184-191) were added to the library. For cloning of the library into the CROPseq-Guide-Puro vector16 (Addgene #86708) using Gibson Assembly, adapter sequences were added to the sgRNA sequences and 74 nucleotide oligos were ordered as an oligo pool (Twist Biosciences). Additional adapters were added to the pooled oligos by PCR (8 cycles, NEB Q5) to generate fragments with a size of 140 nucleotides that were purified (QIAGEN MinElute PCR Purification) before being used for Gibson Assembly. The vector was digested with BsmBl (NEB), size-selected using agarose gel electrophoresis and gel purified (QIAGEN QIAquick Gel Extraction Kit) followed by an additional column purification (QIAGEN QIAquick PCR Purification Kit). 4 Gibson Assembly reactions (10 µl NEBuilder HiFi DNA Assembly, 60 ng vector, 10 ng insert) were prepared and incubated at 50°C for 45 minutes. Reactions were pooled and purified (QIAGEN MinElute PCR Purification) before being used for transformation in Lucigen Endura electrocompetent bacteria (four reactions, 25 µl each). Bacteria were plated on four 245 x 245 x 25mm Bioassay dishes and dilution plates (1:10,000) and incubated at 32°C for 16h. Cells were scraped off the plates and plasmid DNA was extracted using multiple QIAGEN Plasmid Plus Midi kits. Library coverage was 211x and was estimated based on the number of colonies on the dilution plates.

### Cloning

The GFP-donor plasmid with the coding sequence of EGFP flanked by generic sgRNA targeting sites, splice acceptor and splice donor sites and 20 amino acid linkers was assembled from 4 fragments using Gibson Assembly to generate a donor plasmid that is similar in design to a previously published donor plasmid that can be used for intron tagging; see Feldman (2019) Cell 179, 787-799 e17. The DNA fragment with a 25 nucleotide overlap to the pUC19 vector and 32 nucleotide overlap to the N-terminus of EGFP was generated from overlapping oligos (Sigma) and comprises a generic sgRNA targeting site that is not present in the human genome (He, 2016,. Nucleic Acids Res 44, e85) followed by a splice acceptor site (Guzzardo, 2017, Sci Rep 7, 16770) and a flexible 20 amino acid glycine-serine linker. This fragment is followed by a fragment with the coding sequence of EGFP without a start or stop codon that was generated by PCR. The third fragment has a 27 nucleotide overlap to the C-terminus of EGFP and a 25 nucleotide overlap to the pUC19 vector and was generated from overlapping oligos (Sigma) and comprises a flexible 20 amino acid glycine-serine linker followed by a splice donor site (Guzzardo, 2017, loc, cit) the generic sgRNA targeting site. The pUC19 vector was linearized by PCR for Gibson Assembly (NEBuilder HiFi DNA Assembly) with the other three fragments.

The pX330 plasmid expressing Cas9 and the generic sgRNA targeting the donor plasmid was generated by digesting pU6-(Bbsl)_CBh-Cas9-T2A-mCherry (Addgene #64324; see also Chu, 2015, Nat Biotechnol 33, 543-8) with Bbsl followed by ligation with an annealed oligo duplex as described before; see, Ran (2013), Nat Protoc 8, 2281-2308. mCherry was replaced with a Blasticidin resistance (BSD) using Gibson Assembly.

### Pooled protein tapping

For the generation of lentiviral particles, HEK293T cells were transiently transfected with the intron-targeting library and packaging plasmids psPAX2, pMD2.G using PEI transfection. After 12h the media was replaced with IMDM supplemented with 10% FBS and P/S. Viral supernatant was collected 48h after transfection and stored at - 80°C. HAP1 cells were transduced with virus and selected with puromycin for three days. Multiplicity of infection (MOI) was 0.2 and transduction was done at a coverage of 500x. After puromycin selection, cells were grown for one day in media without puromycin before being seeded for transfection (8 million cells per 15 cm dish, 48 million cells in total). One day after seeding, each dish was co-transfected with 20 µg pX330 expressing Cas9-BSD and the generic sgRNA and 10 µg EGFP donor plasmid with 90 µl Turbofection in 2.5 ml OptiMEM as described by the manufacturer. Transfection efficiency was approximately 10% as determined by a transfection done in parallel with pX330 Cas9-mCherry and the EGFP donor plasmid using the same ratio. The next day, cells were subjected to a transient selection using Blasticidin (10 µg/ml) for 24h. After selection, cells were maintained in full media without Blasticidin and sorted five days after transfection by flow cytometry using a Sony Cell Sorter SH800ZD. 0.03% cells were GFP-positive and in total 24,300 of those GFP-positive cells were sorted and the cell population was expanded for 7 days before DNA was isolated to determine sgRNA abundance in the cell population.

### NGS sequencing

In order to generate an NGS library, genomic DNA from one million cells of the GFP positive cell population was isolated and the sgRNA region was amplified by PCR (two reactions using 500 ng genomic DNA, NEB Q5 high-fidelity Polymerase). Illumina adapter ligation and sequencing were done by a commercial sequencing service. To determine sgRNA abundance, sgRNA sequences were extracted from NGS reads using Cutadapt and sgRNA read counts were determined using the MAGeCK count function to match the extracted reads to the sgRNA library. Of the 14,049 sgRNA in the library we considered 1,777 as highly enriched as these sgRNAs accounted for 90% of the obtained sequencing reads while the majority of sgRNAs was not detectable anymore. The remaining 10% of sequencing reads comprise an additional 1,622 sgRNAs, which we do not consider as enriched, as each of them is only supported by a few sequencing reads that might be the result of cells being transduced with two sgRNAs or the result of off-target integration and expression of the GFP-tag. Our library also includes 75 nontargeting sgRNAs making up 0.53% of the sgRNAs in our library. As expected, they are depleted in the pool of GFP-positive, making up 0.15% of the sequencing reads with only 3 non-targeting sgRNAs among the 1,777 sgRNAs we consider enriched.

### Isolation, imaging and sequencing of clonal cell lines

To obtain clonal cell lines, cells were seeded at a concentration of 0.7 cells per well in 96-well cell culture plates. After 9 days of clonal expansion, 768 colonies were harvested using trypsin and cell suspensions were transferred in equal amounts to eight 96-well imaging plates (Perkin Elmer CellCarrier Ultra) and eight corresponding 96-well cell culture plates. After 24h, cells on the imaging plates were imaged on a Perkin Elmer Opera Phenix High Content Screening System (5 fields of view per well, 63x water-immersion objective, confocal mode, excitation: 488 nm, emission: nm, 700 ms). Images were processed using Cell Profiler. To identify the intron-targeting sgRNAs expressed in imaged cells, multiplexed amplicon sequencing of the sgRNA regions was performed in the corresponding clones on the eight 96-well cell culture plates. Cells were lysed and cell lysates were used for PCR to amplify the sgRNA region in each clone using barcoded primers flanking the sgRNA region (36 different 5-mers added to the 5'end of the forward primer and 24 different 5-mers added to the 5'end of the reverse primer, 768 of all possible 864 combinations were used). PCR reactions were pooled and column purified before being send for sequencing by a commercial sequencing service. NGS reads were demultiplexed using Cutadapt (see Martin, M..EMBnet.journal, [S.I.], v. 17, n. 1, p. pp. 10-12, may 2011) and sgRNA read counts for each individual well were obtained using MAGeCK (see, Li (2014) Genome Biol 15, 554 (2014).. For further analysis clones were excluded, for which either no cells in any of the 5 fields of view that were imaged were observed, no sequencing reads for the corresponding well were observed or for which polyclonal cell populations as determined by imaging or detection of multiple sgRNAs per well were observed. Using that strategy, images of 335 clones were obtained for which the expressed intron-targeting sgRNA corresponding to the tagged protein could be identified.

### Comparison of subcellular localization to The Human Protein Atlas

Comparison of subcellular protein localizations of GFP-tagged protein in 335 clones to the localization patterns as annotated on The Human Protein Atlas was done as described previously for the comparison of N- or C-terminally GFP-tagged proteins to IF-based annotations on the Human Protein Atlas, see Stadler (2013) Nat Methods 10, 315-23. Briefly, the overlap was defined as 'identical' if one or multiple main and additional localizations were the same in the intron-tagged clone compared to The Human Protein Atlas, 'similar' if one localization is the same in the clone compared to The Human Protein Atlas with additional localization(s) observed either in the clone or on The Human Protein atlas or 'dissimilar' if there were no common subcellular localization patterns. Extended localization annotations such as nucleoplasm, nuclear speckles or nucleoli that were considered as "nuclear" were not taken into account.

### Live Cell imaging

Live cell imaging was performed on a PerkinElmer Opera Phenix microscope with excitation laser 488 nm, and emission filter 500-550 nm, 700 ms.

### In situ Sequencing

Identification of the expressed sgRNAs by in situ sequencing was performed by following and modifying published protocols, see, e.g., Feldman (2019) loc. cit; Ke (2013) Nat Methods 10, 857-60; and Larsson (2010) Nat Methods 7, 395-7.

After live-cell imaging after treatment with MTX or dBET6, cells were fixed with 4% paraformaldehyde for 30 minutes, washed with PBS, permeabilized with 70% ethanol for 30 minutes and washed with PBS-T (PBS + 0.05% Tween-20) twice. Reverse transcription mix (1x RevertAid RT buffer, 250 µM dNTPs, 0.2 mg/mL BSA, 1 µM RT primer, 0.8 U/mL Ribolock RNase inhibitor, and 4.8 U/mL RevertAid H minus reverse transcriptase) was added to the sample and incubated for 16 hours at 37°C. Following reverse transcription, cells were washed 5 times with PBS-T and post-fixed with 3% paraformaldehyde and 0.1% glutaraldehyde for 30 minutes at room temperature and washed 5 times with PBS-T. Cells were incubated in a padlock probe and extension-ligation reaction mix (1x Ampligase buffer, 0.4 U/mL RNase H, 0.2 mg/mL BSA, 100 nM padlock probe, 0.02 U/mL KlenTaq polymerase, 0.5 U/mL Ampligase and 50 nM dNTPs) for 5 minutes at 37°C and 90 minutes at 45°C, and then washed 2 times with PBS-T. Circularized padlocks were amplified with rolling circle amplification mix (1x Phi29 buffer, 250 µM dNTPs, 0.2 mg/mL BSA, 5% glycerol, and 1 U/mL Phi29 DNA polymerase) at 30°C for 4 hours. Rolling circle amplicons were prepared for sequencing by hybridizing a mix containing sequencing primer oSBS_CROP-seq (1 µM primer in 2X SSC + 10% formamide) for 30 minutes at room temperature. Barcodes were read out using sequencing-by-synthesis reagents from the Illumina NextSeq 500/550 kit v2 (Illumina 15057934). First, samples were washed with incorporation buffer (NextSeq 500/550 buffer cartridge, position 35) and incubated for 4 minutes in incorporation mix (NextSeq 500/550 reagent cartridge, position 31) at 60°C. Samples were then washed with incorporation buffer (4 washes, 60°C for 4 minutes at the last wash) and placed in scan mix (NextSeq 500/550 reagent cartridge, position 30) for imaging. Imaging was performed on a PerkinElmer Opera Phenix microscope with excitation laser: 561 nm, emission filter:570-630, 500 ms; excitation laser: 640 nm, emission filter: 650-760 nm, 500 ms using a 63x water immersion objective, confocal mode. Based were detected as follows: Base T: signal in 561 channel; Base C: signal in 640 channel, Base A: (weaker) signal in both channels, Base G: no signal. Following each imaging cycle, samples were washed with the cleavage mix (NextSeq 500/550 reagent cartridge, position 29) once followed by incubation with cleavage mix for 4 minutes at 60°C to remove dye terminators. Samples were washed 5 times with incorporation buffer before starting the next cycle.

### Primer Sequences employed:

NGS Sequencing
CROP-seq sgRNA amplicon fwd ATCTTGTGGAAAGGACGAAACACC (SEQ ID NO:1)
CROP-seq sgRNA amplicon rev tgtctcaagatctagttacgcca (SEQ ID NO:2)

in situ sequencing
oRT_CROPseq
G+AC+TA+GC+CT+TA+TT+TTAACTTGCTAT (Feldman et al.) (SEQ ID NO:3) oPD_CROPseq

/5Phos/gttttagagctagaaatagcaagCTCCTGTTCGACACCTACCCACCTCATCCCACT
CTTCAaaaggacgaaacaccg (Feldman et al.) (SEQ ID NO:4)

oSBS_CROPseq
CACCTCATCCCACTCTTCAaaaggacgaaacaccg (SEQ ID NO:5) Feldman et al. multiplexed amplicon sequencing barcodes

| | |
|---|---|
| FWD1_1 | GAGAAATCTTGTGGAAAGGACGAAACAC (SEQ ID NO:6) |
| FWD1_2 | CAAGAATCTTGTGGAAAGGACGAAACAC (SEQ ID NO:7) |
| FWD1_3 | GAACAATCTTGTGGAAAGGACGAAACAC (SEQ ID NO:8) |
| FWD1_4 | CCATAATCTTGTGGAAAGGACGAAACAC (SEQ ID NO:9) |
| FWD1_5 | GTTAGATCTTGTGGAAAGGACGAAACAC (SEQ ID NO:10) |
| FWD1_6 | ACTCGATCTTGTGGAAAGGACGAAACAC (SEQ ID NO:11) |
| FWD1_7 | TGTTGATCTTGTGGAAAGGACGAAACAC (SEQ ID NO:12) |
| FWD1_8 | AGGTCATCTTGTGGAAAGGACGAAACAC (SEQ ID NO:13) |
| FWD1_9 | AGGAAATCTTGTGGAAAGGACGAAACAC (SEQ ID NO:14) |
| FWD1_10 | ACAGAATCTTGTGGAAAGGACGAAACAC (SEQ ID NO:15) |
| FWD1_11 | AGACAATCTTGTGGAAAGGACGAAACAC (SEQ ID NO:16) |
| FWD1_12 | TGGTAATCTTGTGGAAAGGACGAAACAC (SEQ ID NO:17) |
| FWD2_1 | CTAGGATCTTGTGGAAAGGACGAAACAC (SEQ ID NO:18) |
| FWD2_2 | CGATGATCTTGTGGAAAGGACGAAACAC (SEQ ID NO:19) |
| FWD2_3 | TTCGCATCTTGTGGAAAGGACGAAACAC (SEQ ID NO:20) |
| FWD2_4 | GGTTCATCTTGTGGAAAGGACGAAACAC (SEQ ID NO:21) |
| FWD2_5 | CCGAAATCTTGTGGAAAGGACGAAACAC (SEQ ID NO:22) |
| FWD2_6 | TCGGAATCTTGTGGAAAGGACGAAACAC (SEQ ID NO:23) |
| FWD2_7 | AAGCAATCTTGTGGAAAGGACGAAACAC (SEQ ID NO:24) |
| FWD2_8 | TCCTAATCTTGTGGAAAGGACGAAACAC (SEQ ID NO:25) |
| FWD2_9 | AATGGATCTTGTGGAAAGGACGAAACAC (SEQ ID NO:26) |
| FWD2_10 | GCATGATCTTGTGGAAAGGACGAAACAC (SEQ ID NO:27) |
| FWD2_11 | TATGCATCTTGTGGAAAGGACGAAACAC (SEQ ID NO:28) |
| FWD2_12 | CCTGTATCTTGTGGAAAGGACGAAACAC (SEQ ID NO:29) |
| FWD3_1 | GTGGAATCTTGTGGAAAGGACGAAACAC (SEQ ID NO:30) |
| FWD3_2 | CTGCAATCTTGTGGAAAGGACGAAACAC (SEQ ID NO:31) |
| FWD3_3 | ACCAAATCTTGTGGAAAGGACGAAACAC (SEQ ID NO:32) |
| FWD3_4 | TGCGAATCTTGTGGAAAGGACGAAACAC (SEQ ID NO:33) |
| FWD3_5 | TGTCAATCTTGTGGAAAGGACGAAACAC (SEQ ID NO:34) |
| FWD3_6 | TAGAGATCTTGTGGAAAGGACGAAACAC (SEQ ID NO:35) |
| FWD3_7 | TACCGATCTTGTGGAAAGGACGAAACAC (SEQ ID NO:36) |
| FWD3_8 | TTGTGATCTTGTGGAAAGGACGAAACAC (SEQ ID NO:37) |
| FWD3_9 | ACACCATCTTGTGGAAAGGACGAAACAC (SEQ ID NO:38) |
| FWD3_10 | ACCTTATCTTGTGGAAAGGACGAAACAC (SEQ ID NO:39) |
| FWD3_11 | CTCTAATCTTGTGGAAAGGACGAAACAC (SEQ ID NO:40) |
| FWD3_12 | TCACGATCTTGTGGAAAGGACGAAACAC (SEQ ID NO:41) |
| REV1_A | GGCAATGTCTCAAGATCTAGTTACGCCA (SEQ ID NO:42) |
| REV1_B | AACGATGTCTCAAGATCTAGTTACGCCA (SEQ ID NO:43) |
| REV1_C | GTCCATGTCTCAAGATCTAGTTACGCCA (SEQ ID NO:44) |
| REV1_D | TGAAGTGTCTCAAGATCTAGTTACGCCA (SEQ ID NO:45) |
| REV1_E | GTACGTGTCTCAAGATCTAGTTACGCCA (SEQ ID NO:46) |
| REV1_F | ACGTGTGTCTCAAGATCTAGTTACGCCA (SEQ ID NO:47) |
| REV1_G | CAACCTGTCTCAAGATCTAGTTACGCCA (SEQ ID NO:48) |
| REV1_H | CACTTTGTCTCAAGATCTAGTTACGCCA (SEQ ID NO:49) |
| REV2_A | CGTAATGTCTCAAGATCTAGTTACGCCA (SEQ ID NO:50) |
| REV2_B | GGTGATGTCTCAAGATCTAGTTACGCCA (SEQ ID NO:51) |
| REV2_C | CCTCATGTCTCAAGATCTAGTTACGCCA (SEQ ID NO:52) |
| REV2_D | ATGAGTGTCTCAAGATCTAGTTACGCCA (SEQ ID NO:53) |
| REV2_E | ATCCGTGTCTCAAGATCTAGTTACGCCA (SEQ ID NO:54) |
| REV2_F | GACTGTGTCTCAAGATCTAGTTACGCCA (SEQ ID NO:55) |
| REV2_G | TCTCCTGTCTCAAGATCTAGTTACGCCA (SEQ ID NO:56) |
| REV2_H | GCTAATGTCTCAAGATCTAGTTACGCCA (SEQ ID NO:57) |
| REV3_A | CTTGATGTCTCAAGATCTAGTTACGCCA (SEQ ID NO:58) |
| REV3_B | GGATATGTCTCAAGATCTAGTTACGCCA (SEQ ID NO:59) |
| REV3_C | AGTAGTGTCTCAAGATCTAGTTACGCCA (SEQ ID NO:60) |
| REV3_D | GATCGTGTCTCAAGATCTAGTTACGCCA (SEQ ID NO:61) |
| REV3_E | AGCTGTGTCTCAAGATCTAGTTACGCCA (SEQ ID NO:62) |
| REV3_F | CTTCCTGTCTCAAGATCTAGTTACGCCA (SEQ ID NO:63) |
| REV3_G | ATTGCTGTCTCAAGATCTAGTTACGCCA (SEQ ID NO:64) |
| REV3_H | GTGCTTGTCTCAAGATCTAGTTACGCCA (SEQ ID NO:65) |

GFP generic donor plasmid (relevant part)

atgttctttcctgcgttatcccctggagatcgagtgccgcatcacCGGCTATTGGTCTTACTGACATCCA
CTTTGCCTTTCTCTCCACAGggtggcggtggctcgggcggtggtgggtccggtggcggcggatctggcg

gtggtggatccgtgagcaagggcgaggagctgttcaccggggtggtgcccatcctggtcgagctggacggcgacgt
aaacggccacaagttcagcgtgtccggcgagggcgagggcgatgccacctacggcaagctgaccctgaagttcat
ctgcaccaccggcaagctgcccgtgccctggcccaccctcgtgaccaccctgacctacggcgtgcagtgcttcagcc
gctaccccgaccacatgaagcagcacgacttcttcaagtccgccatgcccgaaggctacgtccaggagcgcaccat
cttcttcaaggacgacggcaactacaagacccgcgccgaggtgaagttcgagggcgacaccctggtgaaccgcat
cgagctgaagggcatcgacttcaaggaggacggcaacatcctggggcacaagctggagtacaactacaacagcc
acaacgtctatatcatggccgacaagcagaagaacggcatcaaggtgaacttcaagatccgccacaacatcgagg
acggcagcgtgcagctcgccgaccactaccagcagaacacccccatcggcgacggccccgtgctgctgcccgac
aaccactacctgagcacccagtccgccctgagcaaagaccccaacgagaagcgcgatcacatggtcctgctggag
ttcgtgaccgccgccgggatcactctcggcatggacgagctgtacaagggcggcggtggatccggtggcggcggat
ctggcggtggaggttcgggtggcggtgggtcgggcaagGTAAGTATCAATTAGAAGACGAATTCC
CGgtgatgcggcactcgatctcGAATTCttacagacaagctgtgaccgtctccgggagctgcatgtgtcagaggtt
ttcaccgtcatcaccgaaacgcgcgagacgaaagggcctcgtgatacgcctatttttataggttaatgtcatgataataa
tgg (SEQ ID NO:66)

### Example 2: Generation of a cell pool comprising protein tagging and being employed for cellular imaging and in situ sequencing- novel means and methods for real time drug testing and/or screening

The present invention relates to the provision of a large cell pool that comprises individual intro-tagged proteins.

As illustrative, non limiting example of the means and methods of the present invention, a pooled GFP (green fluorescent protein)- intron-tagging of metabolic enzymes is provided herein. As provided herein, a CRISPR/Cas9 mediated intron tagging approach is employed to generate a large pool of cells herein with more than 900 tagged proteins, wherein each cell comprises one tagged protein, i.e. a "one protein per cell" approach is provided. The inventive means and methods of the present invention offer the following advantages, namely that (i) by designing the sgRNA target genes can be chosen as desired (ii) by designing the sgRNA different introns for the same genes can be chosen, allowing to avoid tagging within functionally important domains and (iii) that very homogenous distributions of cells can be generated with roughly equal numbers of clones for each targeted protein.

A second key aspect of the inventive method is the application of *in situ* sequencing. Following exposure of the inventive cell pool to molecules to be screened (for example. drugs and/or pharmacologically relevant molecules), some cells respond with changes in protein localization or in protein abundance (measured by fluorescence microscopy of the GFP tag fused to the protein). The application of a CROP-seq vector as provided and illustrated herein for the intron-targeting sgRNA library allows for in situ sequencing in order to identify the tagged intron. In order to render this compatible with the provided illustrative GFP tagged cell pool, the in situ sequencing protocol was adopted to a two color system

Accordingly, a CRISPR-Cas9 based intron tagging is employed herein to generate cell pools expressing hundreds of labeled/tagged-fusion proteins at endogenous levels, to monitor drug effects on protein levels and/or to localization by time-lapse microscopy. Furthermore, within the means and methods of the present invention is the identification of targeted introns by in situ sequencing. Accordingly, the means and methods of the present invention provide for a pooled protein tagging approach allowing for the localization and even (expression) levels of hundreds of proteins in individual cells in real time; see also illustrative Figure 2a

In context of the present invention, 2,889 genes were selected to be targeted comprising all classic metabolic enzymes and epigenetic modifiers; see Corcoran (2017). Am J Physiol Renal Physiol 312, F533-F542; Birsoy (2015), Cell 162, 540-51. For the 2,387 genes from this set that harbor targetable introns in the selected reading frame, a library comprising 14,049 sgRNAs targeting 11,614 introns (Figure 2b) was designed. To generate a pool of GFP-tagged cells, HAP1 cells were transduced with that sgRNA library followed by co-transfection with a GFP donor plasmid and a plasmid expressing Cas9 and the donor-targeting sgRNA. It was enriched for transfected cells using blasticidin for 24 h and sorted GFP-positive cells 6 days after transfection (Fig. 2c). NGS-based sgRNA amplicon sequencing of the pool of GFP-positive cells identified 1,777 sgRNAs targeting 1,650 introns of 953 genes as highly enriched in the GFP-positive cell pool (Fig. 2d). Compared to genes for which intron targeting sgRNAs did not result in isolation of GFP positive cells, successfully targeted genes have higher average expression in HAP1 cells (Fig. 2e). Clonal cell lines were then isolated from the pool by single cell dilution and clonal expansion, in which GFP localization was imaged (Fig. 2f) and sgRNAs were identified indicating the tagged proteins by an NGS-based multiplex sgRNA amplicon sequencing strategy. After removing cell lines in which more than one sgRNA was present, 362 clonal lines were obtained. The main localization of GFP-tagged proteins in the majority of our cell lines was either cytoplasmic, nuclear or mitochondrial, with some proteins showing a typical ER localization pattern. These localization corresponded to the antibody-based annotations in the Human Protein Atlas (Thul, 2017, Science 356) in 72% of the clones (Fig. 2f), and for 40 GFP-tagged proteins in the clonal cell lines no previous localization data is available.

It was reasoned that the highly diverse pool of cells expressing GFP-tagged proteins can be used to identify compounds that change protein levels or localization of any of the tagged proteins. Therefore, the cell pool was treated with the BRD4-targeting PROTAC dBET6 (Winter (2017). Mol Cell 67, 5-18 e19) and high-content live cell imaging was used to track protein dynamics of GFP-tagged proteins over 9 hours in approximately 7,000 cells in a single well on a 384-well plate (Fig. 6a). A drastic loss of GFP signal was observed in selected clones already 1 h after compound treatment. These clones had a nuclear GFP localization pattern with few selected foci, compatible with the known phase separation behavior of BRD4 (Fig. 6b); see also Sabari (2018) Science 361. The application of the CROP-seq vector16 that expresses the sgRNA sequence in a polyadenylated mRNA transcript enables the cell-specific identification of the targeted intron by situ sequencing (see also Feldman, D. et al. Cell 179, 787-799 e17 (2019), Ke, R. et al. Nat Methods 10, 857-60 (2013), Larsson, C. et al. Nat Methods 7, 395-7 (2010). 17-19). To map targeted introns, the cell pool was fixed and a modified in situ sequencing protocol was developed to identify the sgRNA sequence integrated into individual cells in the pool. Based on the library diversity, eight cycles of nucleotide incorporation and imaging are sufficient to unambiguously assign sgRNA sequences (Fig. 6c). Application of this protocol to the cell pool confirmed that in clones with drastic loss of signal GFP was indeed targeted to BRD4 (Fig. 6d). Analysis of the entire cell pool revealed several other effects of the compound, including the loss of subnuclear localization patterns of MEAF1 and FUBP3, gain of nuclear foci of AKAP8 and SFPQ, and loss of nuclear intensity for UNG (Fig. 6e), none of which are identifiable by global proteomics profiling; see also Winter (2017) ', loc. cit..

It was then tested whether the cell pool also reveals complex cellular responses to compounds that act by conventional mechanisms. Therefore the cell pool was treated with methotrexate (MTX), an antimetabolite impairing DNA and RNA synthesis and causing DNA damage by inhibiting tetrahydrofolate metabolism. Changes to the localizations of several proteins were observed in the cell pool (Fig. 10a), which could be further validated by applying MTX to the arrayed individual clones (Fig. 10b). Importantly, many of the findings are consistent with the known effects of the drug. For example, 24 h MTX treatment caused increased nuclear localization of GFP-tagged ACLY, a metabolic enzyme that has been shown to translocate to the nucleus in response to DNA damage; Sivanand (2017) Mol Cell 67, 252-265 e6. In cell lines expressing either GFP-tagged RPA1 or RPA2, which are part of a heterotrimeric DNA single-strand binding complex, the formation of nuclear foci was observed in response to treatment, presumably by the recruitment of the proteins to sites of DNA damage; see Raderschall (1999), Proc Natl Acad Sci U S A 96, 1921-6. In addition to these predicted effects, a gain of nuclear signal for TPI1, NUDT21 and the transcription factor DR1, the disappearance of nuclear DNMT1 foci, and decreases of RUVBL1 and PADI1 protein was also noted. Importantly, some of the observations are supported by multiple clonal cell lines expressing the same GFP-tagged protein tagged at different intronic sites or by multiple proteins that are part of the same complex, increasing the confidence in the observed drug effects on different proteins.

The generation of targeted GFP tagged cell pools enables, inter alia, the identification of cellular drug responses by time lapse microscopy. Future applications of the present invention and corresponding uses, including deep learning and image recognition as well as direct in situ sequencing, will further accelerate the assignment of the targeted clones directly from screening well. Importantly, the low cost and fast timescales of imaging-based approaches enable applications both in large scale screening and in the rapid optimization of doses and response kinetics in a cellular system. This approach is especially useful for the discovery and development of PROTACs and molecular glue degraders, for which activity can easily be determined by the disappearance of the tagged protein, however we document herein also that the means and methods of the present invention can be employed to verify and/or confirm known drug actions and/or to discover new effects of known drugs. Importantly, intron tagging can easily be applied for other sets of genes beyond metabolic enzymes and potentially in a genome-wide manner to study protein dynamics at scale not only in response to drug treatment or other physiological perturbations.

### Example 3 - Minicircle plasmids

For protein tagging with an intron tagging strategy, a generic sgRNA is excising a fluorescent tag flanked by splice acceptor and donor sites from a generic donor plasmid. This excision was done by cutting the donor plasmid twice, resulting in the fragment containing the coding sequence of the tag flanked by splice acceptor and a splice donor (Fig. 11A). However, another fragment containing the plasmid backbone is generated and this fragment is equally likely getting integrated at target sites as specified by an intron targeting sgRNA. If the plasmid backbone gets integrated, these target sites are not available anymore for integration of GFP containing plasmid, thereby lowering the editing efficiency (Fig. 11B). Furthermore, it was observed that in some cases the donor plasmid is cut only once, leading to integration of the entire linearized plasmid containing not only the coding sequence of the protein tag, but also the plasmid backbone containing sequences for plasmid amplification on bacteria (origin of replication, resistance gene etc., Fig. 11C). While cells with those integrations would still be GFP positive, it was observed that integration of the plasmid backbone can change levels of or correct localization of GFP-fusions. Here it is shown that using a minicircle that contains only the coding sequence of the protein tag flanked by splice acceptor and donor sites and has only one generic sgRNA target site to linearize the minicircle, significantly improves the intron tagging strategy by addressing both disadvantages of the current strategy (Fig. 11D).

To compare the conventional donor plasmid to a minicircle, it was attempted to tag CANX at intron 14 by using either a GFP donor plasmid containing two generic sgRNA sites or a GFP minicircle containing only one generic sgRNA sites and no plasmid backbone. A tagging rate of 3,0% was achieved when using the GFP donor plasmid as determined by analyzing transfected cells by flow cytometry (Fig. 12A). When using the GFP minicircle, the tagging rate increased approximately two-fold to 6,5% (Fig. 12B). To rule out that the observed increase is only resulting from a higher number of transfected fragments (GFP minicircle is 1 kb in length compared to the 3 kb GFP donor plasmid, which is why 200 ng of GFP minicircle contains three times as many molecules as 200 ng of GFP donor plasmid) a third sample was transfected with 1/3 the amount of donor plasmid (67 ng) and again an improved tagging rate of 5,7% was observed (Fig 12C). Therefore, it was concluded that the improved tagging rate is not simply the result of a higher number of fragments that are present in the cell, but is due to the design of the minicircle, making integration of any plasmid backbone fragment impossible. Additionally, when using the minicircle it is not possible to integrate the GFP containing fragment together with the plasmid backbone as observed when the conventional donor plasmid is only cut once and linearized plasmid gets integrated. These integrations can result in lower expression levels of tagged genes and indeed less GFP-positive cells with intensity levels only slightly above the autofluorescence of cells were observed (note the very distinct GFP-positive population in the two minicircle samples compared to the population in the GFP donor plasmid sample).

In a second independent experiment, similar improvements when using the minicircle were observed (4-fold increase in GFP-positive cells when using the same amount of GFP minicircle DNA as GFP donor plasmid and 3-fold when using 1/3 the amount of minicircle DNA) but in this experiment the overall tagging rates were lower due to lower transfection efficiency (less than 10% of cells that were analyzed were transfected, Fig. 13A-C).

Minicircle DNA was produced with a commercial minicircle production kit (SBI MC-Easy^{™} Minicircle DNA Production Kit). First, a parental production plasmid was generated by cloning a DNA fragment starting with the generic sgRNA target site followed by a splice acceptor, a 20 amino acid linker sequence, the coding sequence of EGFP, another 20 amino acid linker sequence and a splice donor site into the pMC.BESPX-MCS1 production plasmid. The DNA fragment was generated by PCR using the GFP donor plasmid as a template, pMC.BESPX-MCS1 was digested with EcoRV and the fragment was integrated at the restriction site via Gibson Assembly. The E. coli producer strain ZYCY10P3S2T was transformed with the ligation reaction and clonal bacterial colonies were selected for isolation and sequencing of parental plasmid. A colony containing the correct parental plasmid was used for minicircle production as described by the manufacturer. In brief, bacteria were grown overnight in the provided growth media and induction media was added the next day to induce *att* recombination and parental plasmid backbone degradation. Minicircle DNA was isolated from bacterial pellets using multiple Qiagen Plasmid Plus Midi kits and the produced minicircle was analyzed by restriction enzyme digest and gel electrophoresis.

For intron tagging experiments, A549 cells were cells seeded in a 12-well plate and were co-transfected with 400 ng of the CROPseq plasmid expressing the intron-targeting sgRNA targeting intron 14 of the CANX gene, 400 ng of the pX330 plasmid expressing Cas9-mCherry and the donor-targeting sgRNA, together with 200 ng of the GFP donor plasmid or 200 ng GFP minicircle using Lipofectamine 3000 as described by the manufacturer. In samples with 1/3 of the amount of GFP minicircle, cells were cotransfected with 467 ng of the CROPseq plasmid with the intron-targeting sgRNA targeting intron 14 of the CANX gene, 467 ng of the pX330 plasmid expressing Cas9-mCherry and the donor-targeting sgRNA, and 67 ng GFP minicircle. To enrich for transfected cells, mCherry-positive cells were sorted 48h after transfection and expanded for one week before GFP-positive cells were sorted. In an independent experiment a px330 plasmid expressing Cas9-BSD instead of Cas9-mCherry was used, cells were not enriched for transfected cells and GFP-positive cells were sorted 48 h after transfection.

Annotation and sequence of the parental GFP minicircle production plasmid:

| | |
|---|---|
| 1-738 | 32 consecutive ISce1 site |
| 782-816 | attB site |
| 852-871 | generic sgRNA target site |
| 872-874 | PAM site |
| 875-916 | splice acceptor |
| 917-976 | 20-amino-acid-linker |
| 977-1690 | EGFP |
| 1691-1753 | 21-amino-acid-linker |
| 1754-1776 | splice donor |
| 1812-1850 | attP site |
| 1937-2731 | Kanamycin resistance |
| 3034-5071 | EcoE1 origin |

Only the sequence between the attB and attP site circularizes and remains in the final GFP minicircle.

Only the part between the attB and attP sites was designed. The parental producer plasmid backbone is part of the commercial SBI MC-Easy^{™} Minicircle DNA Production Kit.

ACATTACCCTGTTATCCCTAGATACATTACCCTGTTATCCCAGATGACATACCCT
GTTATCCCTAGATGACATTACCCTGTTATCCCAGATGACATTACCCTGTTATCCC
TAGATACATTACCCTGTTATCCCAGATGACATACCCTGTTATCCCTAGATGACAT
TACCCTGTTATCCCAGATGACATTACCCTGTTATCCCTAGATACATTACCCTGTT
ATCCCAGATGACATACCCTGTTATCCCTAGATGACATTACCCTGTTATCCCAGAT
GACATTACCCTGTTATCCCTAGATACATTACCCTGTTATCCCAGATGACATACCC
TGTTATCCCTAGATGACATTACCCTGTTATCCCAGATGACATTACCCTGTTATCC
CTAGATACATTACCCTGTTATCCCAGATGACATACCCTGTTATCCCTAGATGACA
TTACCCTGTTATCCCAGATGACATTACCCTGTTATCCCTAGATACATTACCCTGT
TATCCCAGATGACATACCCTGTTATCCCTAGATGACATTACCCTGTTATCCCAGA
TGACATTACCCTGTTATCCCTAGATACATTACCCTGTTATCCCAGATGACATACC
CTGTTATCCCTAGATGACATTACCCTGTTATCCCAGATGACATTACCCTGTTATC
CCTAGATACATTACCCTGTTATCCCAGATGACATACCCTGTTATCCCTAGATGAC
ATTACCCTGTTATCCCAGATAAACTCAATGATGATGATGATGATGGTCGAGACTC
AGCGGCCGCGGTGCCAGGGCGTGCCCTTGGGCTCCCCGGGCGCGACTAGTGA
ATTCAGATCTGATCCTGCGTTATCCCCTGGAGATCGAGTGCCGCATCACCGGCT
ATTGGTCTTACTGACATCCACTTTGCCTTTCTCTCCACAGGGTGGCGGTGGCTC
GGGCGGTGGTGGGTCCGGTGGCGGCGGATCTGGCGGTGGTGGATCCGTGAG
CAAGGGCGAGGAGCTGTTCACCGGGGTGGTGCCCATCCTGGTCGAGCTGGAC
GGCGACGTAAACGGCCACAAGTTCAGCGTGTCCGGCGAGGGCGAGGGCGATG
CCACCTACGGCAAGCTGACCCTGAAGTTCATCTGCACCACCGGCAAGCTGCCC
GTGCCCTGGCCCACCCTCGTGACCACCCTGACCTACGGCGTGCAGTGCTTCAG

CCGCTACCCCGACCACATGAAGCAGCACGACTTCTTCAAGTCCGCCATGCCCG
AAGGCTACGTCCAGGAGCGCACCATCTTCTTCAAGGACGACGGCAACTACAAG
ACCCGCGCCGAGGTGAAGTTCGAGGGCGACACCCTGGTGAACCGCATCGAGC
TGAAGGGCATCGACTTCAAGGAGGACGGCAACATCCTGGGGCACAAGCTGGAG
TACAACTACAACAGCCACAACGTCTATATCATGGCCGACAAGCAGAAGAACGGC
ATCAAGGTGAACTTCAAGATCCGCCACAACATCGAGGACGGCAGCGTGCAGCT
CGCCGACCACTACCAGCAGAACACCCCCATCGGCGACGGCCCCGTGCTGCTG
CCCGACAACCACTACCTGAGCACCCAGTCCGCCCTGAGCAAAGACCCCAACGA
GAAGCGCGATCACATGGTCCTGCTGGAGTTCGTGACCGCCGCCGGGATCACTC
TCGGCATGGACGAGCTGTACAAGGGCGGCGGTGGATCCGGTGGCGGCGGATC
TGGCGGTGGAGGTTCGGGTGGCGGTGGGTCGGGCAAGGTAAGTATCAATTAGA
AGACGAATTCCATCTCTAGAGTCGACCCATGGGGGCCCGCCCCAACTGGGGTA
ACCTTTGAGTTCTCTCAGTTGGGGGTAATCAGCATCATGATGTGGTACCACATC
ATGATGCTGATTATAAGAATGCGGCCGCCACACTCTAGTGGATCTCGAGTTAAT
AATTCAGAAGAACTCGTCAAGAAGGCGATAGAAGGCGATGCGCTGCGAATCGG
GAGCGGCGATACCGTAAAGCACGAGGAAGCGGTCAGCCCATTCGCCGCCAAG
CTCTTCAGCAATATCACGGGTAGCCAACGCTATGTCCTGATAGCGGTCCGCCAC
ACCCAGCCGGCCACAGTCGATGAATCCAGAAAAGCGGCCATTTTCCACCATGAT
ATTCGGCAAGCAGGCATCGCCATGGGTCACGACGAGATCCTCGCCGTCGGGCA
TGCTCGCCTTGAGCCTGGCGAACAGTTCGGCTGGCGCGAGCCCCTGATGCTCT
TCGTCCAGATCATCCTGATCGACAAGACCGGCTTCCATCCGAGTACGTGCTCGC
TCGATGCGATGTTTCGCTTGGTGGTCGAATGGGCAGGTAGCCGGATCAAGCGT
ATGCAGCCGCCGCATTGCATCAGCCATGATGGATACTTTCTCGGCAGGAGCAA
GGTGAGATGACAGGAGATCCTGCCCCGGCACTTCGCCCAATAGCAGCCAGTCC
CTTCCCGCTTCAGTGACAACGTCGAGCACAGCTGCGCAAGGAACGCCCGTCGT
GGCCAGCCACGATAGCCGCGCTGCCTCGTCTTGCAGTTCATTCAGGGCACCGG
ACAGGTCGGTCTTGACAAAAAGAACCGGGCGCCCCTGCGCTGACAGCCGGAAC
ACGGCGGCATCAGAGCAGCCGATTGTCTGTTGTGCCCAGTCATAGCCGAATAG
CCTCTCCACCCAAGCGGCCGGAGAACCTGCGTGCAATCCATCTTGTTCAATCAT
GCGAAACGATCCTCATCCTGTCTCTTGATCAGAGCTTGATCCCCTGCGCCATCA
GATCCTTGGCGGCGAGAAAGCCATCCAGTTTACTTTGCAGGGCTTCCCAACCTT
ACCAGAGGGCGCCCCAGCTGGCAATTCCGGTTCGCTTGCTGTCCATAAAACCG
CCCAGTCTAGCTATCGCCATGTAAGCCCACTGCAAGCTACCTGCTTTCTCTTTG

CGCTTGCGTTTTCCCTTGTCCAGATAGCCCAGTAGCTGACATTCATCCGGGGTC
AGCACCGTTTCTGCGGACTGGCTTTCTACGTGCTCGAGGGGGGCCAAACGGTC
TCCAGCTTGGCTGTTTTGGCGGATGAGAGAAGATTTTCAGCCTGATACAGATTA
AATCAGAACGCAGAAGCGGTCTGATAAAACAGAATTTGCCTGGCGGCAGTAGC
GCGGTGGTCCCACCTGACCCCATGCCGAACTCAGAAGTGAAACGCCGTAGCGC
CGATGGTAGTGTGGGGTCTCCCCATGCGAGAGTAGGGAACTGCCAGGCATCAA
ATAAAACGAAAGGCTCAGTCGAAAGACTGGGCCTTTCGTTTTATCTGTTGTTTGT
CGGTGAACGCTCTCCTGAGTAGGACAAATCCGCCGGGAGCGGATTTGAACGTT
GCGAAGCAACGGCCCGGAGGGTGGCGGGCAGGACGCCCGCCATAAACTGCCA
GGCATCAAATTAAGCAGAAGGCCATCCTGACGGATGGCCTTTTTGCGTTTCTAC
AAACTCTTTTGTTTATTTTTCTAAATACATTCAAATATGTATCCGCTCATGACCAAA
ATCCCTTAACGTGAGTTTTCGTTCCACTGAGCGTCAGACCCCGTAGAAAAGATC
AAAGGATCTTCTTGAGATCCTTTTTTTCTGCGCGTAATCTGCTGCTTGCAAACAA
AAAAACCACCGCTACCAGCGGTGGTTTGTTTGCCGGATCAAGAGCTACCAACTC
TTTTTCCGAAGGTAACTGGCTTCAGCAGAGCGCAGATACCAAATACTGTCCTTCT
AGTGTAGCCGTAGTTAGGCCACCACTTCAAGAACTCTGTAGCACCGCCTACATA
CCTCGCTCTGCTAATCCTGTTACCAGTGGCTGCTGCCAGTGGCGATAAGTCGTG
TCTTACCGGGTTGGACTCAAGACGATAGTTACCGGATAAGGCGCAGCGGTCGG
GCTGAACGGGGGGTTCGTGCACACAGCCCAGCTTGGAGCGAACGACCTACACC
GAACTGAGATACCTACAGCGTGAGCTATGAGAAAGCGCCACGCTTCCCGAAGG
GAGAAAGGCGGACAGGTATCCGGTAAGCGGCAGGGTCGGAACAGGAGAGCGC
ACGAGGGAGCTTCCAGGGGGAAACGCCTGGTATCTTTATAGTCCTGTCGGGTTT
CGCCACCTCTGACTTGAGCGTCGATTTTTGTGATGCTCGTCAGGGGGGCGGAG
CCTATGGAAAAACGCCAGCAACGCGGCCTTTTTACGGTTCCTGGCCTTTTGCTG
GCCTTTTGCTCACATGTTCTTTCCTGCGTTATCCCCTGATTCTGTGGATAACCGT
ATTACCGCCTTTGAGTGAGCTGATACCGCTCGCCGCAGCCGAACGACCGAGCG
CAGCGAGTCAGTGAGCGAGGAAGCGGAAGAGCGCCTGATGCGGTATTTTCTCC
TTACGCATCTGTGCGGTATTTCACACCGCATATGGTGCACTCTCAGTACAATCTG
CTCTGATGCCGCATAGTTAAGCCAGTATACACTCCGCTATCGCTACGTGACTGG
GTCATGGCTGCGCCCCGACACCCGCCAACACCCGCTGACGCGCCCTGACGGG
CTTGTCTGCTCCCGGCATCCGCTTACAGACAAGCTGTGACCGTCTCCGGGAGC
TGCATGTGTCAGAGGTTTTCACCGTCATCACCGAAACGCGCGAGGCAGCAGAT
CAATTCGCGCGCGAAGGCGAAGCGGCATGCATAATGTGCCTGTCAAATGGACG

AAGCAGGGATTCTGCAAACCCTATGCTACTCCGTCAAGCCGTCAATTGTCTGAT
TCGTTACCAATTATGACAACTTGACGGCTACATCATTCACTTTTTCTTCACAACC
GGCACGGAACTCGCTCGGGCTGGCCCCGGTGCATTTTTTAAATACCCGCGAGA
AATAGAGTTGATCGTCAAAACCAACATTGCGACCGACGGTGGCGATAGGCATCC
GGGTGGTGCTCAAAAGCAGCTTCGCCTGGCTGATACGTTGGTCCTCGCGCCAG
CTTAAGACGCTAATCCCTAACTGCTGGCGGAAAAGATGTGACAGACGCGACGG
CGACAAGCAAACATGCTGTGCGACGCTGGCGAT (SEQ ID NO:67)

## Claims

1. A method for monitoring the effect of an environmental factor on the proteome or parts thereof of a cell, the method comprising the steps of:
(a) selecting introns to be targeted in the genome of a cell;
(b) identifying guide RNA (gRNA) sequences suitable for inserting a tag in the selected introns in the genome of the cell;
(c) cloning identified gRNA sequences and tag sequence into vectors;
(d) contacting a population of the cell with said vectors of (c) to integrate the tag of (b) into selected introns;
(e) exposing cell population to environmental factor; and
(f) monitoring the effect of the environmental factor on the proteome based on the detection of the tag prior to exposure of the cell population to the environmental factor and subsequent to exposure of the cell population to the environmental factor
wherein in step (c) the identified gRNA sequences are cloned into a transduction vector and the tag sequence is cloned into a minicircle DNA.

2. The method of claim 1, further comprising a sequencing step subsequent to step (d).

3. The method of claim 2, wherein the sequencing step is subsequent to step (f).

4. The method of claim 2 or 3, wherein the gRNA insert, or a part thereof, of (a) cell(s) of the population is sequenced in the genome of said cell(s) or in the transcriptome of said cell(s).

5. The method of any one of claims 1 to 4, wherein the transduction vector is a sequencing vector, preferably a Crop-Seq vector.

6. The method of any one of claims 1 to 5, wherein the introns to be targeted are comprised in genomic sequences of metabolic enzymes, chromatin proteins, kinases, genes coding for proteins in the ubiquitin/proteasome pathways, transcription factors, ion channels, transporters, receptors or wherein at least one intron per protein coding gene in the genome is targeted.

7. The method of any one of claims 1 to 6, wherein the introns to be targeted are selected based on the reading frame of the upstream exonic sequence, wherein the sequence to be inserted is in-frame with the exonic sequence.

8. The method of any one of claims 1 to 7, wherein the gRNA sequences suitable for inserting a tag in the selected introns in the genome of the cell are identified according to cas9 cutting efficiency or Cpf1 cutting efficiency or Cas12b cutting efficiency.

9. The method of any one of claims 1 to 8, wherein the gRNA sequences suitable for inserting a tag in the selected introns in the genome of the cell are identified according to their occurrence in the genome of the cell, preferably wherein the occurrence is 1.

10. The method of any one of claims 1 to 90, wherein the gRNA is a single gRNA (sgRNA).

11. The method of any one of claims 1 to 10, wherein the environmental factor is selected from radiation, a chemical compound, a biological compound, temperature, nutrient depletion, ion concentration(s).

12. The method of any one of claims 1 to 11, wherein the effect on the proteome is selected from protein expression, protein localization, surface expression, protein-protein interaction, protein stability, protein mobility.

13. The method of any one of claims 1 to 12, wherein the cell is a HAP1 cell, K562 cell, HeLa cell, KBM7 cell, BT474 cell, MG-63 cell, SKNAS cell, A427 cell, A375 cell, A498 cell, RCH-ACV cell, HEK293T cell, A673 cell, SK-N-MC cell, A549 cell, SKMES1 cell, NCIH727 cell, THP1 cell, NB4 cell, MOLM13 cell, KASUMI-1 cell, HEL cell, NB-4 cell, HL-60 cell, RS4-11 cell, MOLT7 cell, aTC1 cell, bTC3 cell, Min6 cell or another cell line, preferentially an adherent, non-migratory cell line.

14. The method of any one of claims 1 to 13, wherein the tag is a fluorescence tag, preferably GFP, EGFP, YFP, RFP, or a tag suitable for detection by covalent (e.g. Halo tag, Clip tag, Snap tag) or non-covalent (e.g. Strep-tag, HA tag, dTag) binding to a detection reagent enabling detection by microscopy, e.g. fluorescence or luminescence.

15. The method of any one of claims 1 to 14, further comprising a step of separating tagged cells from non-tagged cells, preferably wherein cells are separated using FACS.

## Patentansprüche

1. Verfahren zur Überwachung der Auswirkung eines Umweltfaktors auf das Proteom einer Zelle oder auf Teile von diesem, wobei das Verfahren die Schritte umfasst:
(a) Auswählen von Introns, die im Genom einer Zelle Target sein sollen;
(b) Identifizieren von Guide-RNA (gRNA)-Sequenzen, die für die Insertion eines Tags in die ausgewählten Introns im Genom der Zelle geeignet sind;
(c) Clonieren der identifizierten gRNA-Sequenzen und der Tag-Sequenz in Vektoren;
(d) Inkontaktbringen einer Population der Zelle mit den Vektoren aus (c), um den Tag aus (b) in ausgewählte Introns zu integrieren;
(e) Aussetzen der Zellpopulation gegenüber einem Umweltfaktor; und
(f) Überwachen der Auswirkung des Umweltfaktors auf das Proteom auf der Grundlage des Nachweises des Tags vor dem Aussetzen der Zellpopulation gegenüber dem Umweltfaktor und nach dem Aussetzen der Zellpopulation gegenüber dem Umweltfaktor;
wobei in Schritt (c) die identifizierten gRNA-Sequenzen in einen Transduktionsvektor cloniert werden und die Tag-Sequenz in eine Minicircle-DNA cloniert wird.

2. Verfahren nach Anspruch 1, das des Weiteren einen Sequenzierungsschritt anschließend an Schritt (d) umfasst.

3. Verfahren nach Anspruch 2, wobei der Sequenzierungsschritt anschließend an Schritt (f) stattfindet.

4. Verfahren nach Anspruch 2 oder 3, wobei das gRNA-Insert, oder ein Teil davon, einer Zelle/von Zellen der Population im Genom der Zelle(n) oder im Transkriptom der Zelle(n) sequenziert wird.

5. Verfahren nach einem der Ansprüche 1 bis 4, wobei der Transduktionsvektor ein Sequenzierungsvektor, bevorzugt ein Crop-Seq-Vektor, ist.

6. Verfahren nach einem der Ansprüche 1 bis 5, wobei die Introns, die das Target sein sollen, in genomischen Sequenzen von Stoffwechselenzymen, Chromatinproteinen, Kinasen, Genen, die Proteine in den Ubiquitin/Proteasom-Wegen codieren, Transkriptionsfaktoren, lonenkanälen, Transportern, Rezeptoren umfasst sind oder wobei mindestens ein Intron pro Protein-codierendem Gen im Genom das Target ist.

7. Verfahren nach einem der Ansprüche 1 bis 6, wobei die Introns, die das Target sein sollen, ausgewählt sind auf der Grundlage des Leserahmens der Exonsequenz stromaufwärts, wobei die zu inserierende Sequenz mit der Exonsequenz im Leserahmen (in-frame) liegt.

8. Verfahren nach einem der Ansprüche 1 bis 7, wobei die gRNA-Sequenzen, die für das Inserieren eines Tags in die ausgewählten Introns im Genom der Zelle geeignet sind, anhand der Cas9-Schneideeffizienz oder Cpf1-Schneideeffizienz oder Cas12b-Schneideeffizienz identifiziert werden.

9. Verfahren nach einem der Ansprüche 1 bis 8, wobei die gRNA-Sequenzen, die für das Inserieren eines Tags in die ausgewählten Introns im Genom der Zelle geeignet sind, anhand ihres Auftretens im Genom der Zelle identifiziert werden, bevorzugt wobei das Auftreten 1 ist.

10. Verfahren nach einem der Ansprüche 1 bis 9, wobei die gRNA eine Single gRNA (sgRNA) ist.

11. Verfahren nach einem der Ansprüche 1 bis 10, wobei der Umweltfaktor ausgewählt ist aus Strahlung, einer chemischen Verbindung, einer biologischen Verbindung, Temperatur, Nährstoffmangel, lonenkonzentration(en).

12. Verfahren nach einem der Ansprüche 1 bis 11, wobei die Auswirkung auf das Proteom ausgewählt ist aus Proteinexpression, Proteinlokalisierung, Oberflächenexpression, Protein-Protein-Interaktion, Proteinstabilität, Proteinmobilität.

13. Verfahren nach einem der Ansprüche 1 bis 12, wobei die Zelle eine HAP1-Zelle, K562-Zelle, HeLa-Zelle, KBM7-Zelle, BT474-Zelle, MG-63-Zelle, SKNAS-Zelle, A427-Zelle, A375-Zelle, A498-Zelle, RCH-ACV-Zelle, HEK293T-Zelle, A673-Zelle, SK-N-MC-Zelle, A549-Zelle, SKMES1-Zelle, NCIH727-Zelle, THP1-Zelle, NB4-Zelle, MOLM13-Zelle, KASUMI-1-Zelle, HEL-Zelle, NB-4-Zelle, HL-60-Zelle, RS4-11-Zelle, MOLT7-Zelle, aTC1-Zelle, bTC3-Zelle, Min6-Zelle oder eine andere Zelllinie, bevorzugt eine adhärente, nicht-migratorische Zellline ist.

14. Verfahren nach einem der Ansprüche 1 bis 13, wobei der Tag ein Fluoreszenz-Tag, bevorzugt GFP, EGFP, YFP, RFP, oder ein Tag ist, der zum Nachweis durch kovalente (z.B. Halo-Tag, Clip-Tag, Snap-Tag) oder nicht-kovalente (z.B. Strep-Tag, HA-Tag, dTag) Bindung an ein Nachweisreagens geeignet ist, das den Nachweis mittels Mikroskopie, z.B. Fluoreszenz oder Lumineszenz, ermöglicht.

15. Verfahren nach einem der Ansprüche 1 bis 14, des Weiteren umfassend einen Schritt des Trennens von getaggten von nicht-getaggten Zellen, bevorzugt wobei die Zellen unter Verwendung von FACS getrennt werden.

## Revendications

1. Procédé de surveillance de l'effet d'un facteur environnemental sur le protéome ou des parties de celui-ci d'une cellule, le procédé comprenant les étapes de :
(a) sélection d'introns à cibler dans le génome d'une cellule ;
(b) identification de séquences d'ARN guide (ARNg) adaptées pour insérer un marqueur dans les introns sélectionnés du génome de la cellule ;
(c) clonage des séquences d'ARNg identifiées et de la séquence de marqueur dans des vecteurs ;
(d) mise en contact d'une population de la cellule avec lesdits vecteurs de (c) pour intégrer le marqueur de (b) dans des introns sélectionnés ;
(e) exposition de la population de cellules au facteur environnemental ; et
(f) surveillance de l'effet du facteur environnemental sur le protéome sur la base de la détection du marqueur avant l'exposition de la population de cellules au facteur environnemental et après l'exposition de la population de cellules au facteur environnemental ;
dans lequel à l'étape (c), les séquences ARNg identifiées sont clonées dans un vecteur de transduction et la séquence de marqueur est clonée dans un minicercle d'ADN.

2. Procédé selon la revendication 1, comprenant en outre une étape de séquençage postérieure à l'étape (d).

3. Procédé selon la revendication 2, dans laquelle l'étape de séquençage est postérieure à l'étape (f).

4. Procédé selon la revendication 2 ou 3, dans lequel l'insert d'ARNg, ou une partie de celui-ci, d'une ou de plusieurs cellules de la population est séquencé dans le génome de ladite ou desdites cellules ou dans le transcriptome de ladite ou desdites cellules.

5. Procédé selon l'une quelconque des revendications 1 à 4, dans lequel le vecteur de transduction est un vecteur de séquençage, de préférence un vecteur Crop-Seq.

6. Procédé selon l'une quelconque des revendications 1 à 5, dans lequel les introns à cibler sont compris dans des séquences génomiques d'enzymes métaboliques, de protéines de la chromatine, de kinases, de gènes codant pour des protéines des voies ubiquitine/protéasome, de facteurs de transcription, de canaux ioniques, de transporteurs, de récepteurs, ou dans lequel au moins un intron par gène codant pour une protéine dans le génome est ciblé.

7. Procédé selon l'une quelconque des revendications 1 à 6, dans lequel les introns à cibler sont sélectionnés sur la base du cadre de lecture de la séquence exonique en amont, dans lequel la séquence à insérer est dans le cadre de la séquence exonique.

8. Procédé selon l'une quelconque des revendications 1 à 7, dans lequel les séquences d'ARNg adaptées pour insérer un marqueur dans les introns sélectionnés du génome de la cellule sont identifiées en fonction de l'efficacité de coupure de Cas9, de l'efficacité de coupure de Cpf1 ou de l'efficacité de coupure de Cas12b.

9. Procédé selon l'une quelconque des revendications 1 à 8, dans lequel les séquences d'ARNg adaptées pour insérer un marqueur dans les introns sélectionnés du génome de la cellule sont identifiées en fonction de leur occurrence dans le génome de la cellule, de préférence dans lequel l'occurrence est de 1.

10. Procédé selon l'une quelconque des revendications 1 à 9, dans lequel l'ARNg est un ARNg simple (ARNgs).

11. Procédé selon l'une quelconque des revendications 1 à 10, dans lequel le facteur environnemental est choisi parmi le rayonnement, un composé chimique, un composé biologique, la température, l'appauvrissement en nutriments, la (les) concentration(s) ionique(s).

12. Procédé selon l'une quelconque des revendications 1 à 11, dans lequel l'effet sur le protéome est choisi parmi l'expression de la protéine, la localisation de la protéine, l'expression de surface, l'interaction protéine-protéine, la stabilité de la protéine, la mobilité de la protéine.

13. Procédé selon l'une quelconque des revendications 1 à 12, dans lequel la cellule est une cellule HAP1, une cellule K562, une cellule HeLa, une cellule KBM7, une cellule BT474, une cellule MG-63, une cellule SKNAS, une cellule A427, une cellule A375, une cellule A498, une cellule RCH-ACV, une cellule HEK293T, une cellule A673, une cellule SK-N-MC, une cellule A549, SKMES1, une cellule NCIH727, une cellule THP1, une cellule NB4, une cellule MOLM13, une cellule KASUMI-1, une cellule HEL, une cellule NB-4, une cellule HL-60, une cellule RS4-11, une cellule MOLT7, une cellule aTC1, une cellule bTC3, une cellule Min6 ou une autre lignée cellulaire, de préférence une lignée cellulaire adhérente et non migratoire.

14. Procédé selon l'une quelconque des revendications 1 à 13, dans lequel le marqueur est un marqueur de fluorescence, de préférence GFP, EGFP, YFP, RFP, ou un marqueur adapté à la détection par liaison covalente (par exemple, marqueur Halo, marqueur Clip, marqueur Snap) ou non covalente (par exemple, marqueur Strep, marqueur HA, marqueur d) à un réactif de détection permettant la détection par microscopie, par exemple, par fluorescence ou luminescence.

15. Procédé selon l'une quelconque des revendications 1 à 14, comprenant en outre une étape de séparation des cellules marquées et des cellules non marquées, de préférence dans lequel les cellules sont séparées à l'aide de la technique FACS.
